# EUROPEAN PATENT APPLICATION

(11) **EP 4 851 190 A1**
(43) Date of publication of application: **09.12.2026**
(21) Application number: 25747603.6
(22) Date of filing: 20.01.2025
(51) Int. Cl.: C07K 16/28, C07K 19/00, A61K 39/395, A61K 47/68, A61P 35/00

(54) **SINGLE-DOMAIN ANTIBODY TARGETING TFR1 AND USE THEREOF**

(30) Priority: 31.01.2024 CN 202410134895
(71) Applicant: Assembly Medicine, LLC., Shanghai, 201203 (CN)
(72) Inventor: PAN, Liqiang, Shanghai 201203 (CN); ZHOU, Liujuan, Shanghai 201203 (CN); BAI, Zhaoshuai, Shanghai 201203 (CN); CHOU, James Jeiwen, Shanghai 201203 (CN)
(74) Representative: Lavoix
(86) International application number: PCT/CN2025/073392
(87) International publication number: WO 2025/162031

(57) **Abstract**

Provided in the present invention are a single-domain antibody targeting TfR1 and the use thereof. Specifically, provided in the present invention are a corresponding anti-TfR1 antibody, a chimeric antigen receptor, a fusion protein, a recombinant protein and a coding nucleic acid thereof, an expression vector, a host cell, etc. In addition, an anti-TfR1 single-domain antibody-PMO conjugate is prepared. The single-domain antibody has properties such as binding activity and stability, and can be used for tumor therapy and immunodetection.

## Description

### TECHNICAL FIELD

The present invention relates to the field of biomedicine or biopharmaceutical technology, and more specifically, to a single domain antibody targeting TfR1 and use thereof.

### BACKGROUND

Transferrin receptor (TfR) is a type II transmembrane glycoprotein receptor composed of two single-pass transmembrane glycoprotein subunits, existing as a homodimer. The extracellular domain (ECD) of each dimer can bind to two transferrin (Tf) molecules, mediating the transport of iron ions from outside the cell to the inside to meet the needs of cell growth and activity. There are two families of TfR, namely TfR1 and TfR2. Studies have shown that TfR1 is expressed at a basal level in most tissues and cells. High expression of TfR1 is generally associated with rapid cell proliferation, including high expression in a variety of highly metastatic tumor tissues, such as brain tumors, nasopharyngeal carcinoma, prostate cancer, breast cancer, ovarian cancer, liver cancer, leukemia, colon cancer, and lung cancer. In addition, high expression of TfR1 is also associated with cellular activities, for example, it is highly expressed on the surface of muscle cells that require large amounts of iron, and on the surface of brain endothelial cells to maintain iron homeostasis in the brain.

Single domain antibodies are a novel type of antibody molecule, discovered by the Belgian immunologist Hamers-Casterman's research group in camelids. Compared to conventional antibodies, they have a smaller molecular weight, about one-tenth the size of traditional IgG antibodies. Therefore, they have a simple structure, are easy to modify, have strong tissue penetration, and low immunogenicity. Moreover, single domain antibodies lack an Fc domain, so they do not mediate antibody-dependent cellular cytotoxicity (ADCC) effects and cause cytotoxicity when used as targeting molecules. In addition, they also have the advantages such as good stability, resistance to high temperature and extreme pH environments, and low production costs. Therefore, single domain antibodies are excellent targeting molecules with great value for targeted drug development.

With the development of immunotherapy, antibody drugs have given rise to various conjugation formats, including antibody drug conjugates (ADCs), antibody oligonucleotide conjugates (AOCs), immune stimulating antibody conjugates (ISACs), etc. By leveraging the targeting properties of antibodies, pharmacologically active molecules such as toxins, antisense nucleic acids, immune receptor agonists, are delivered to the vicinity of target sites, thereby improving the utilization of the pharmacologically active molecules and reducing side effects. Compared to conjugates prepared with traditional antibody fragments such as IgG, Fab, and scFv, single-domain antibody conjugates offer simpler conjugation processes, enhanced tissue penetration, and improved therapeutic efficacy *in vivo,* thanks to their smaller molecular size and greater stability. Furthermore, compared to similarly small-sized cell-penetrating peptide conjugates like peptide-PMOs, single-domain antibody conjugates exhibit superior targeting capabilities, demonstrating distinct advantages.

Based on the above background, there is an urgent need in the art to develop a single domain antibody targeting TfR1 and to develop corresponding anti-tumor drugs, and drugs targeting muscle and brain diseases.

### SUMMARY OF THE INVENTION

The objective of the present invention is to provide a single domain antibody targeting TfR1 and use thereof.

In a first aspect of the present invention, there is provided an anti-TfR1 single domain antibody, wherein the anti-TfR1 single domain antibody has one or more complementarity determining regions (CDRs) selected from the group consisting of:
(1) CDR1 as set forth in SEQ ID NO: 51, CDR2 as set forth in SEQ ID NO: 73, and CDR3 as set forth in SEQ ID NO: 98;
(2) CDR1 as set forth in SEQ ID NO: 54, CDR2 as set forth in SEQ ID NO: 61, and CDR3 as set forth in SEQ ID NO: 53;
(3) CDR1 as set forth in SEQ ID NO: 54, CDR2 as set forth in SEQ ID NO: 61, and CDR3 as set forth in SEQ ID NO: 56;
(4) CDR1 as set forth in SEQ ID NO: 60, CDR2 as set forth in SEQ ID NO: 67, and CDR3 as set forth in SEQ ID NO: 71;
(5) CDR1 as set forth in SEQ ID NO: 69, CDR2 as set forth in SEQ ID NO: 64, and CDR3 as set forth in SEQ ID NO: 74;
(6) CDR1 as set forth in SEQ ID NO: 45, CDR2 as set forth in SEQ ID NO: 49, and CDR3 as set forth in SEQ ID NO: 50;
(7) CDR1 as set forth in SEQ ID NO: 66, CDR2 as set forth in SEQ ID NO: 116, and CDR3 as set forth in SEQ ID NO: 119;
(8) CDR1 as set forth in SEQ ID NO: 45, CDR2 as set forth in SEQ ID NO: 100, and CDR3 as set forth in SEQ ID NO: 50;
(9) CDR1 as set forth in SEQ ID NO: 51, CDR2 as set forth in SEQ ID NO: 46, and CDR3 as set forth in SEQ ID NO: 118;
(10) CDR1 as set forth in SEQ ID NO: 45, CDR2 as set forth in SEQ ID NO: 46, and CDR3 as set forth in SEQ ID NO: 47;
(11) CDR1 as set forth in SEQ ID NO: 57, CDR2 as set forth in SEQ ID NO: 76, and CDR3 as set forth in SEQ ID NO: 77;
(12) CDR1 as set forth in SEQ ID NO: 81, CDR2 as set forth in SEQ ID NO: 79, and CDR3 as set forth in SEQ ID NO: 80;
(13) CDR1 as set forth in SEQ ID NO: 96, CDR2 as set forth in SEQ ID NO: 70, and CDR3 as set forth in SEQ ID NO: 83;
(14) CDR1 as set forth in SEQ ID NO: 48, CDR2 as set forth in SEQ ID NO: 49, and CDR3 as set forth in SEQ ID NO: 86;
(15) CDR1 as set forth in SEQ ID NO: 93, CDR2 as set forth in SEQ ID NO: 82, and CDR3 as set forth in SEQ ID NO: 89;
(16) CDR1 as set forth in SEQ ID NO: 75, CDR2 as set forth in SEQ ID NO: 58, and CDR3 as set forth in SEQ ID NO: 68;
(17) CDR1 as set forth in SEQ ID NO: 48, CDR2 as set forth in SEQ ID NO: 49, and CDR3 as set forth in SEQ ID NO: 92;
(18) CDR1 as set forth in SEQ ID NO: 111, CDR2 as set forth in SEQ ID NO: 85, and CDR3 as set forth in SEQ ID NO: 62;
(19) CDR1 as set forth in SEQ ID NO: 75, CDR2 as set forth in SEQ ID NO: 58, and CDR3 as set forth in SEQ ID NO: 95;
(20) CDR1 as set forth in SEQ ID NO: 45, CDR2 as set forth in SEQ ID NO: 52, and CDR3 as set forth in SEQ ID NO: 47;
(21) CDR1 as set forth in SEQ ID NO: 63, CDR2 as set forth in SEQ ID NO: 55, and CDR3 as set forth in SEQ ID NO: 65;
(22) CDR1 as set forth in SEQ ID NO: 108, CDR2 as set forth in SEQ ID NO: 88, and CDR3 as set forth in SEQ ID NO: 101;
(23) CDR1 as set forth in SEQ ID NO: 87, CDR2 as set forth in SEQ ID NO: 91, and CDR3 as set forth in SEQ ID NO: 62;
(24) CDR1 as set forth in SEQ ID NO: 90, CDR2 as set forth in SEQ ID NO: 94, and CDR3 as set forth in SEQ ID NO: 104;
(25) CDR1 as set forth in SEQ ID NO: 102, CDR2 as set forth in SEQ ID NO: 97, and CDR3 as set forth in SEQ ID NO: 59;
(26) CDR1 as set forth in SEQ ID NO: 84, CDR2 as set forth in SEQ ID NO: 103, and CDR3 as set forth in SEQ ID NO: 107;
(27) CDR1 as set forth in SEQ ID NO: 99, CDR2 as set forth in SEQ ID NO: 106, and CDR3 as set forth in SEQ ID NO: 110;
(28) CDR1 as set forth in SEQ ID NO: 105, CDR2 as set forth in SEQ ID NO: 109, and CDR3 as set forth in SEQ ID NO: 113;
(29) CDR1 as set forth in SEQ ID NO: 78, CDR2 as set forth in SEQ ID NO: 58, and CDR3 as set forth in SEQ ID NO: 68;
(30) CDR1 as set forth in SEQ ID NO: 96, CDR2 as set forth in SEQ ID NO: 112, and CDR3 as set forth in SEQ ID NO: 115;
(31) CDR1 as set forth in SEQ ID NO: 48, CDR2 as set forth in SEQ ID NO: 114, and CDR3 as set forth in SEQ ID NO: 117;
(32) CDR1 as set forth in SEQ ID NO: 72, CDR2 as set forth in SEQ ID NO: 64, and CDR3 as set forth in SEQ ID NO: 74.

In another preferred embodiment, the complementarity determining regions (CDRs) of the anti-TfR1 single domain antibody are defined according to the IMGT numbering scheme.

In another preferred embodiment, any one of the above amino acid sequences further comprises a derivative sequence obtained by optionally adding, deleting, modifying, and/or substituting at least one (e.g., 1 to 3, preferably 1 to 2, and more preferably 1) amino acid, while retaining the ability to specifically bind to TfR1.

In another preferred embodiment, the derivative sequence, which is obtained by adding, deleting, modifying, and/or substituting at least one amino acid and retains the ability to specifically bind to TfR1, is an amino acid sequence having a homology or sequence identity of at least 85%, at least 90%, at least 95%, at least 96%, at least 97%, at least 98%, or at least 99%.

In another preferred embodiment, the CDR1, CDR2, and CDR3 are flanked by the framework regions FR1, FR2, FR3, and FR4 of a VHH chain, respectively.

In another preferred embodiment, the anti-TfR1 single domain antibody has the structure shown in Formula (I):

FR1-CDR1-FR2-CDR2-FR3-CDR3-FR4 Formula (I)

wherein each "-" is independently a linker peptide or a peptide bond.

In another preferred embodiment, there is an inter-loop disulfide bond between the CDR1 and CDR3 regions of the anti-TfR1 single domain antibody.

In another preferred embodiment, the anti-TfR1 single domain antibody further comprises framework regions (FRs).

In another preferred embodiment, the framework regions (FRs) comprise human FR regions, murine, rabbit, monkey, alpaca, or camel FR regions.

In another preferred embodiment, the framework regions (FRs) are one or more selected from the group consisting of:
(1) FR1 as set forth in SEQ ID NO: 133, FR2 as set forth in SEQ ID NO: 134, FR3 as set forth in SEQ ID NO: 135, and FR4 as set forth in SEQ ID NO: 136;
(2) FR1 as set forth in SEQ ID NO: 137, FR2 as set forth in SEQ ID NO: 138, FR3 as set forth in SEQ ID NO: 139, and FR4 as set forth in SEQ ID NO: 140;
(3) FR1 as set forth in SEQ ID NO: 141, FR2 as set forth in SEQ ID NO: 138, FR3 as set forth in SEQ ID NO: 139, and FR4 as set forth in SEQ ID NO: 140;
(4) FR1 as set forth in SEQ ID NO: 141, FR2 as set forth in SEQ ID NO: 167, FR3 as set forth in SEQ ID NO: 168, and FR4 as set forth in SEQ ID NO: 136;
(5) FR1 as set forth in SEQ ID NO: 158, FR2 as set forth in SEQ ID NO: 151, FR3 as set forth in SEQ ID NO: 152, and FR4 as set forth in SEQ ID NO: 159;
(6) FR1 as set forth in SEQ ID NO: 120, FR2 as set forth in SEQ ID NO: 162, FR3 as set forth in SEQ ID NO: 150, and FR4 as set forth in SEQ ID NO: 136;
(7) FR1 as set forth in SEQ ID NO: 206, FR2 as set forth in SEQ ID NO: 207, FR3 as set forth in SEQ ID NO: 208, and FR4 as set forth in SEQ ID NO: 127;
(8) FR1 as set forth in SEQ ID NO: 133, FR2 as set forth in SEQ ID NO: 134, FR3 as set forth in SEQ ID NO: 183, and FR4 as set forth in SEQ ID NO: 136;
(9) FR1 as set forth in SEQ ID NO: 120, FR2 as set forth in SEQ ID NO: 134, FR3 as set forth in SEQ ID NO: 135, and FR4 as set forth in SEQ ID NO: 136;
(10) FR1 as set forth in SEQ ID NO: 141, FR2 as set forth in SEQ ID NO: 134, FR3 as set forth in SEQ ID NO: 160, and FR4 as set forth in SEQ ID NO: 136;
(11) FR1 as set forth in SEQ ID NO: 120, FR2 as set forth in SEQ ID NO: 121, FR3 as set forth in SEQ ID NO: 122, and FR4 as set forth in SEQ ID NO: 123;
(12) FR1 as set forth in SEQ ID NO: 124, FR2 as set forth in SEQ ID NO: 125, FR3 as set forth in SEQ ID NO: 126, and FR4 as set forth in SEQ ID NO: 127;
(13) FR1 as set forth in SEQ ID NO: 128, FR2 as set forth in SEQ ID NO: 129, FR3 as set forth in SEQ ID NO: 130, and FR4 as set forth in SEQ ID NO: 127;
(14) FR1 as set forth in SEQ ID NO: 131, FR2 as set forth in SEQ ID NO: 125, FR3 as set forth in SEQ ID NO: 132, and FR4 as set forth in SEQ ID NO: 127;
(15) FR1 as set forth in SEQ ID NO: 142, FR2 as set forth in SEQ ID NO: 143, FR3 as set forth in SEQ ID NO: 144, and FR4 as set forth in SEQ ID NO: 127;
(16) FR1 as set forth in SEQ ID NO: 145, FR2 as set forth in SEQ ID NO: 146, FR3 as set forth in SEQ ID NO: 147, and FR4 as set forth in SEQ ID NO: 148;
(17) FR1 as set forth in SEQ ID NO: 141, FR2 as set forth in SEQ ID NO: 149, FR3 as set forth in SEQ ID NO: 150, and FR4 as set forth in SEQ ID NO: 127;
(18) FR1 as set forth in SEQ ID NO: 128, FR2 as set forth in SEQ ID NO: 151, FR3 as set forth in SEQ ID NO: 152, and FR4 as set forth in SEQ ID NO: 153;
(19) FR1 as set forth in SEQ ID NO: 154, FR2 as set forth in SEQ ID NO: 155, FR3 as set forth in SEQ ID NO: 156, and FR4 as set forth in SEQ ID NO: 127;
(20) FR1 as set forth in SEQ ID NO: 157, FR2 as set forth in SEQ ID NO: 146, FR3 as set forth in SEQ ID NO: 147, and FR4 as set forth in SEQ ID NO: 148;
(21) FR1 as set forth in SEQ ID NO: 161, FR2 as set forth in SEQ ID NO: 134, FR3 as set forth in SEQ ID NO: 135, and FR4 as set forth in SEQ ID NO: 136;
(22) FR1 as set forth in SEQ ID NO: 163, FR2 as set forth in SEQ ID NO: 134, FR3 as set forth in SEQ ID NO: 135, and FR4 as set forth in SEQ ID NO: 136;
(23) FR1 as set forth in SEQ ID NO: 141, FR2 as set forth in SEQ ID NO: 138, FR3 as set forth in SEQ ID NO: 139, and FR4 as set forth in SEQ ID NO: 164;
(24) FR1 as set forth in SEQ ID NO: 142, FR2 as set forth in SEQ ID NO: 165, FR3 as set forth in SEQ ID NO: 166, and FR4 as set forth in SEQ ID NO: 127;
(25) FR1 as set forth in SEQ ID NO: 169, FR2 as set forth in SEQ ID NO: 170, FR3 as set forth in SEQ ID NO: 171, and FR4 as set forth in SEQ ID NO: 172;
(26) FR1 as set forth in SEQ ID NO: 173, FR2 as set forth in SEQ ID NO: 174, FR3 as set forth in SEQ ID NO: 175, and FR4 as set forth in SEQ ID NO: 127;
(27) FR1 as set forth in SEQ ID NO: 176, FR2 as set forth in SEQ ID NO: 177, FR3 as set forth in SEQ ID NO: 178, and FR4 as set forth in SEQ ID NO: 179;
(28) FR1 as set forth in SEQ ID NO: 180, FR2 as set forth in SEQ ID NO: 181, FR3 as set forth in SEQ ID NO: 182, and FR4 as set forth in SEQ ID NO: 127;
(29) FR1 as set forth in SEQ ID NO: 163, FR2 as set forth in SEQ ID NO: 162, FR3 as set forth in SEQ ID NO: 150, and FR4 as set forth in SEQ ID NO: 136;
(30) FR1 as set forth in SEQ ID NO: 184, FR2 as set forth in SEQ ID NO: 185, FR3 as set forth in SEQ ID NO: 186, and FR4 as set forth in SEQ ID NO: 127;
(31) FR1 as set forth in SEQ ID NO: 187, FR2 as set forth in SEQ ID NO: 146, FR3 as set forth in SEQ ID NO: 147, and FR4 as set forth in SEQ ID NO: 148;
(32) FR1 as set forth in SEQ ID NO: 188, FR2 as set forth in SEQ ID NO: 151, FR3 as set forth in SEQ ID NO: 152, and FR4 as set forth in SEQ ID NO: 159;
(33) FR1 as set forth in SEQ ID NO: 189, FR2 as set forth in SEQ ID NO: 190, FR3 as set forth in SEQ ID NO: 191, and FR4 as set forth in SEQ ID NO: 127;
(34) FR1 as set forth in SEQ ID NO: 120, FR2 as set forth in SEQ ID NO: 192, FR3 as set forth in SEQ ID NO: 193, and FR4 as set forth in SEQ ID NO: 140;
(35) FR1 as set forth in SEQ ID NO: 194, FR2 as set forth in SEQ ID NO: 195, FR3 as set forth in SEQ ID NO: 196, and FR4 as set forth in SEQ ID NO: 127;
(36) FR1 as set forth in SEQ ID NO: 197, FR2 as set forth in SEQ ID NO: 198, FR3 as set forth in SEQ ID NO: 147, and FR4 as set forth in SEQ ID NO: 148;
(37) FR1 as set forth in SEQ ID NO: 163, FR2 as set forth in SEQ ID NO: 138, FR3 as set forth in SEQ ID NO: 199, and FR4 as set forth in SEQ ID NO: 140;
(38) FR1 as set forth in SEQ ID NO: 200, FR2 as set forth in SEQ ID NO: 201, FR3 as set forth in SEQ ID NO: 202, and FR4 as set forth in SEQ ID NO: 127;
(39) FR1 as set forth in SEQ ID NO: 120, FR2 as set forth in SEQ ID NO: 203, FR3 as set forth in SEQ ID NO: 204, and FR4 as set forth in SEQ ID NO: 127;
(40) FR1 as set forth in SEQ ID NO: 205, FR2 as set forth in SEQ ID NO: 151, FR3 as set forth in SEQ ID NO: 152, and FR4 as set forth in SEQ ID NO: 159;
(41) FR1 as set forth in SEQ ID NO: 209, FR2 as set forth in SEQ ID NO: 167, FR3 as set forth in SEQ ID NO: 168, and FR4 as set forth in SEQ ID NO: 136;
(42) FR1 as set forth in SEQ ID NO: 128, FR2 as set forth in SEQ ID NO: 165, FR3 as set forth in SEQ ID NO: 166, and FR4 as set forth in SEQ ID NO: 127;
(43) FR1 as set forth in SEQ ID NO: 133, FR2 as set forth in SEQ ID NO: 134, FR3 as set forth in SEQ ID NO: 135, and FR4 as set forth in SEQ ID NO: 148;
(44) FR1 as set forth in SEQ ID NO: 133, FR2 as set forth in SEQ ID NO: 134, FR3 as set forth in SEQ ID NO: 216, and FR4 as set forth in SEQ ID NO: 127;
(45) FR1 as set forth in SEQ ID NO: 133, FR2 as set forth in SEQ ID NO: 134, FR3 as set forth in SEQ ID NO: 216, and FR4 as set forth in SEQ ID NO: 217;
(46) FR1 as set forth in SEQ ID NO: 133, FR2 as set forth in SEQ ID NO: 134, FR3 as set forth in SEQ ID NO: 216, and FR4 as set forth in SEQ ID NO: 148;
(47) FR1 as set forth in SEQ ID NO: 141, FR2 as set forth in SEQ ID NO: 134, FR3 as set forth in SEQ ID NO: 216, and FR4 as set forth in SEQ ID NO: 217;
(48) FR1 as set forth in SEQ ID NO: 141, FR2 as set forth in SEQ ID NO: 134, FR3 as set forth in SEQ ID NO: 216, and FR4 as set forth in SEQ ID NO: 148.

In another preferred embodiment, the amino acid sequence of the VHH chain of the anti-TfR1 single domain antibody is selected from one or more of SEQ ID NOs: 1-44 and 210-215.

In another preferred embodiment, the anti-TfR1 single domain antibody comprises a humanized antibody, an animal-derived antibody, or a chimeric antibody.

In another preferred embodiment, the animal is a non-human mammal, preferably a mouse, goat, rabbit, monkey, alpaca, or camel.

In another preferred embodiment, the anti-TfR1 single domain antibody is a partially or fully humanized antibody.

In another preferred embodiment, the anti-TfR1 single domain antibody has one or more characteristics selected from the group consisting of:
(a) specifically binding to human and/or alpaca TfR1;
(b) specifically binding to monkey TfR1;
(c) not binding or weakly binding to murine TfR1;
(d) not binding or weakly binding to monkey TfR1;
(e) blocking the binding of TfR1 to transferrin;
(f) not blocking the binding of TfR1 to transferrin;
(g) not binding to TfR2; and/or
(h) not blocking the binding of TfR1 to HFE protein.

In another preferred embodiment, the monkey is a rhesus monkey.

In a second aspect of the present invention, there is provided an anti-TfR1 antibody, wherein the antibody comprises one or more VHH chains of the anti-TfR1 single domain antibody of the first aspect of the present invention.

In another preferred embodiment, the amino acid sequence of the VHH chain of the anti-TfR1 single domain antibody is selected from one or more of SEQ ID NOs: 1-44 and 210-215.

In another preferred embodiment, the anti-TfR1 antibody may be a monomer, a bivalent antibody, and/or a multivalent antibody.

In another preferred embodiment, the anti-TfR1 antibody is a bivalent antibody.

In another preferred embodiment, the anti-TfR1 antibody comprises a humanized antibody, an animal-derived antibody, or a chimeric antibody.

In another preferred embodiment, the animal is a non-human mammal, preferably a mouse, goat, rabbit, monkey, alpaca, or camel.

In another preferred embodiment, the anti-TfR1 antibody is a partially or fully humanized antibody.

In another preferred embodiment, the anti-TfR1 antibody has one or more characteristics selected from the group consisting of:
(a) specifically binding to human and/or alpaca TfR1;
(b) specifically binding to monkey TfR1;
(c) not binding or weakly binding to murine TfR1;
(d) not binding or weakly binding to monkey TfR1;
(e) blocking the binding of TfR1 to transferrin;
(f) not blocking the binding of TfR1 to transferrin;
(g) not binding to TfR2; and/or
(h) not blocking the binding of TfR1 to HFE protein.

In a third aspect of the present invention, there is provided a chimeric antigen receptor (CAR), wherein the CAR comprises an extracellular domain, and the extracellular domain comprises the anti-TfR1 single domain antibody of the first aspect of the present invention, or the anti-TfR1 antibody of the second aspect of the present invention.

In another preferred embodiment, the extracellular domain further comprises a signal peptide.

In another preferred embodiment, the extracellular domain further comprises other exogenous proteins.

In another preferred embodiment, the CAR has the structure shown in Formula Ia:

L-Nb-H-TM-C-CD3ζ (Ia)

wherein,
L is absent or a signal peptide sequence;
Nb is a specific binding domain;
H is absent or a hinge region;
TM is a transmembrane domain;
C is a costimulatory signaling domain;
CD3ζ is a cytoplasmic signaling sequence derived from CD3ζ (including wild-type, or mutants/modified forms thereof);
"-" is a linker peptide or a peptide bond.

In another preferred embodiment, L is respectively a signal peptide of proteins selected from the group consisting of: CD8, GM-CSF, CD4, CD28, CD137, or mutants/modified forms thereof, and combinations thereof.

In another preferred embodiment, Nb targets TfR1.

In another preferred embodiment, Nb is an anti-TfR1 single domain antibody.

In another preferred embodiment, H is a hinge region of proteins selected from the group consisting of: CD8, CD28, CD137, IgG, and combinations thereof.

In another preferred embodiment, H is a human IgG1 Fc hinge region.

In another preferred embodiment, TM is a transmembrane region of proteins selected from the group consisting of: CD28, CD3 epsilon, CD45, CD4, CD5, CD8, CD9, CD16, CD22, CD33, CD37, CD64, CD80, CD86, CD134, CD137, CD154, CD278, CD152, CD279, CD233, or mutants/modified forms thereof, and combinations thereof.

In another preferred embodiment, C is a costimulatory domain of proteins selected from the group consisting of: OX40, CD2, CD7, CD27, CD28, CD30, CD40, CD70, CD134, 4-1BB (CD137), PD-1, Dap10, LIGHT, NKG2C, B7-H3, ICAM-1, LFA-1 (CD11a/CD18), ICOS (CD278), NKG2D, GITR, OX40L, 2B4, TLR, or mutants/modified forms thereof, and combinations thereof.

In a fourth aspect of the present invention, there is provided a multispecific antibody, wherein a first antigen-binding region of the multispecific antibody comprises the anti-TfR1 single domain antibody of the first aspect of the present invention, or the anti-TfR1 antibody of the second aspect of the present invention.

In another preferred embodiment, the multispecific antibody further comprises a second antigen-binding region targeting a target selected from the group consisting of: BCMA, CD28, CD73, GPC3, HER2, PMSA, 4-1BB, OX40, GLP-1, Trop2, FGL1, LFA-3, 2B4, 5T4, α-4 integrin, α-V integrin, α4β7 integrin, α4β7 integrin, α-SMA, AGR2, Apelin J receptor, APRIL, B7-H3, B7-H4, BAFF, BTLA, C5 complement, C-242, CA9, CA19-9, carbonic anhydrase 9, CD2, CD3, CD6, CD9, CD11a, CD11b, CD11c, CD19, CD20, CD22, CD24, CD25, CD27, CD30, CD33, CD38, CD40, CD40L, CD41, CD44, CD44v6, CD47, CD51, CD52, CD56, CD64, CD69, CD70, CD71, CD74, CD80, CD81, CD86, CD95, CD107a, CD117, CD123, CD125, CD132 (IL-2Rg), CD133, CD137, CD138, CD160, CD166, CD172A, CD248, CEACAM5 (CEA), CEACAM6 (NCA-90), CLAUDIN-3, CLAUDIN-4, cMet, collagen, Cripto, CSFR, CSFR-1, CTLA-4, CTGF, CXCL10, CXCL13, CXCR1, CXCR2, CXCR4, CYR61, DL44, DLK1, DLL4, DPP-4, DSG1, EDA, EDB, EGFR, EGFRviii, endothelin B receptor (ETBR), ENPP3, EpCAM, EPHA2, EPHB2, ERBB3, RSV F protein, FAP, FGF-2, FGF8, FGFR1, FGFR2, FGFR3, FGFR4, FLT-3, folate receptor α (FRα), FSP-1, GAL3ST1, G-CSF, G-CSFR, GD2, GITR, GLUT1, GLUT4, GM-CSF, GM-CSFR, GP IIb/IIIa receptor, Gpl30, GPIIB/IIIA, GPNMB, GRP78, HER2/neu, HER3, HER4, HGF, hGH, HLA-DR, HVEM, hyaluronidase, ICOS, IFNα, IFNβ, IFNγ, IgE, IgE receptor (FceRI), IGF, IGF1R, IL1B, IL1R, IL2, IL11, IL12, IL12p40, IL-12R, IL-12Rβl, IL13, IL13R, IL13Ra2, IL15, IL17, IL18, IL21, IL23, IL23R, IL27/IL27R (wsxl), IL29, IL-31R, IL31/IL31R, IL2R, IL4, IL4R, IL6, IL6R, IL1 receptor accessory protein (IL1RAP), insulin receptor, Jagged ligands, Jagged 1, Jagged 2, KISS1-R, KLRG1, LAG-3, LIF-R, Lewis X, LIGHT, LRP4, LRRC26, Ly6G6D, LyPD1, MCSP, mesothelin, MRP4, MUC1, mucin-16 (MUC16, CA-125), Na/K ATPase, NGF, Nicastrin, Notch receptors, Notch 1, Notch 2, Notch 3, Notch 4, NOV, OSM-R, OX-40, PAR2, PDGF-AA, PDGF-BB, PDGFRα, PDGFRβ, PD-1, PD-L1, PD-L2, phosphatidylserine, P1GF, PSCA, PSMA, PSGR, RAAG12, RAGE, SLC44A4, Siglec15, STEAP1, STEAP2, TAG-72, TAPA1, TEM-8, TGFβ, TIGIT, TIM-3, TLR2, TLR4, TLR6, TLR7, TLR8, TLR9, TMEM31, TNFα, TNFR, TNFRS12A, TRAIL-R1, TRAIL-R2, TRK-A, TRK-B, uPAR, VAP1, VCAM-1, VEGF, VEGF-A, VEGF-B, VEGF-C, VEGF-D, VEGFR1, VEGFR2, VEGFR3, VISTA, WISP-1, WISP-2, WISP-3, and combinations thereof.

In another preferred embodiment, the multispecific antibody comprises one or more second antigen-binding regions.

In another preferred embodiment, the second antigen-binding region is a single domain antibody.

In another preferred embodiment, the multispecific antibody further comprises an Fc region of an antibody.

In another preferred embodiment, the antigen-binding region is an antibody or antibody fragment, and the antibody fragment includes: (i) a Fab fragment; (ii) a F(ab')2 fragment; (iii) a Fd fragment; (iv) a Fv fragment; (v) a single-chain Fv (scFv) molecule; (vi) a dAb fragment.

In a fifth aspect of the present invention, there is provided a recombinant protein, wherein the recombinant protein has:
(i) the anti-TfR1 single domain antibody of the first aspect of the present invention, the anti-TfR1 antibody of the second aspect of the present invention, or an active fragment thereof;
(ii) optionally, a polypeptide molecule or fragment with therapeutic function; and/or
(iii) optionally, a functional domain that improves the physicochemical properties or drugability of the protein.

In another preferred embodiment, improving the physicochemical properties or drugability of the protein includes extending the half-life of the anti-TfR1 single domain antibody.

In another preferred embodiment, the functional domain that improves the physicochemical properties or drugability of the protein includes an Fc region or human serum albumin (HSA).

In another preferred embodiment, the recombinant protein further comprises (iv) optionally, a tag sequence that facilitates expression and/or purification.

In another preferred embodiment, the tag includes an Fc tag, HA tag, GGGS sequence, FLAG tag, Myc tag, 6His tag, or combinations thereof.

In another preferred embodiment, the recombinant protein specifically binds to TfR1.

In another preferred embodiment, the recombinant protein is a monomer, dimer, or multimer.

In another preferred embodiment, the tag is an Fc tag.

In another preferred embodiment, the polypeptide molecule or fragment with therapeutic function includes, but is not limited to: polypeptide molecules or fragments targeting BCMA, CD28, CD73, GPC3, HER2, PMSA, 4-1BB, OX40, GLP-1, Trop2, FGL1, LFA-3, 2B4, 5T4, α-4 integrin, α-V integrin, α4β7 integrin, α4β7 integrin, α-SMA, AGR2, Apelin J receptor, APRIL, B7-H3, B7-H4, BAFF, BTLA, C5 complement, C-242, CA9, CA19-9, carbonic anhydrase 9, CD2, CD3, CD6, CD9, CD11a, CD11b, CD11c, CD19, CD20, CD22, CD24, CD25, CD27, CD30, CD33, CD38, CD40, CD40L, CD41, CD44, CD44v6, CD47, CD51, CD52, CD56, CD64, CD69, CD70, CD71, CD74, CD80, CD81, CD86, CD95, CD107a, CD117, CD123, CD125, CD132 (IL-2Rg), CD133, CD137, CD138, CD160, CD166, CD172A, CD248, CEACAM5 (CEA), CEACAM6 (NCA-90), CLAUDIN-3, CLAUDIN-4, cMet, collagen, Cripto, CSFR, CSFR-1, CTLA-4, CTGF, CXCL10, CXCL13, CXCR1, CXCR2, CXCR4, CYR61, DL44, DLK1, DLL4, DPP-4, DSG1, EDA, EDB, EGFR, EGFRviii, endothelin B receptor (ETBR), ENPP3, EpCAM, EPHA2, EPHB2, ERBB3, RSV F protein, FAP, FGF-2, FGF8, FGFR1, FGFR2, FGFR3, FGFR4, FLT-3, folate receptor α (FRα), FSP-1, GAL3ST1, G-CSF, G-CSFR, GD2, GITR, GLUT1, GLUT4, GM-CSF, GM-CSFR, GP IIb/IIIa receptor, Gpl30, GPIIB/IIIA, GPNMB, GRP78, HER2/neu, HER3, HER4, HGF, hGH, HLA-DR, HVEM, hyaluronidase, ICOS, IFNα, IFNβ, IFNγ, IgE, IgE receptor (FceRI), IGF, IGF1R, IL1B, IL1R, IL2, IL11, IL12, IL12p40, IL-12R, IL-12Rβl, IL13, IL13R, IL13Ra2, IL15, IL17, IL18, IL21, IL23, IL23R, IL27/IL27R (wsxl), IL29, IL-31R, IL31/IL31R, IL2R, IL4, IL4R, IL6, IL6R, IL1 receptor accessory protein (IL1RAP), insulin receptor, Jagged ligands, Jagged 1, Jagged 2, KISS1-R, KLRG1, LAG-3, LIF-R, Lewis X, LIGHT, LRP4, LRRC26, Ly6G6D, LyPD1, MCSP, mesothelin, MRP4, MUC1, mucin-16 (MUC16, CA-125), Na/K ATPase, NGF, Nicastrin, Notch receptors, Notch 1, Notch 2, Notch 3, Notch 4, NOV, OSM-R, OX-40, PAR2, PDGF-AA, PDGF-BB, PDGFRα, PDGFRβ, PD-1, PD-L1, PD-L2, phosphatidylserine, P1GF, PSCA, PSMA, PSGR, RAAG12, RAGE, SLC44A4, Siglec15, STEAP1, STEAP2, TAG-72, TAPA1, TEM-8, TGFβ, TIGIT, TIM-3, TLR2, TLR4, TLR6, TLR7, TLR8, TLR9, TMEM31, TNFα, TNFR, TNFRS12A, TRAIL-R1, TRAIL-R2, TRK-A, TRK-B, uPAR, VAP1, VCAM-1, VEGF, VEGF-A, VEGF-B, VEGF-C, VEGF-D, VEGFR1, VEGFR2, VEGFR3, VISTA, WISP-1, WISP-2, WISP-3, or combinations thereof.

In another preferred embodiment, the polypeptide molecule or fragment with therapeutic function includes, but is not limited to: insulin, IL-2, interferon, calcitonin, GHRH peptide, intestinal peptide analogs, albumin, antibody fragments, cytokines.

In another preferred embodiment, the recombinant protein comprises a fusion protein.

In another preferred embodiment, the fusion protein has the following elements from N-terminus to C-terminus:

A-B;

wherein element A is an anti-TfR1 single domain antibody; element B is an Fc region or human serum albumin (HSA);
"-" represents a peptide bond.

In another preferred embodiment, the amino acid sequence of the VHH chain of the anti-TfR1 single domain antibody is selected from one or more of SEQ ID NOs: 1-50.

In another preferred embodiment, the Fc region is a human IgG Fc region (e.g., human IgG1 Fc region).

In a sixth aspect of the present invention, there is provided a polynucleotide, wherein the polynucleotide encodes a protein selected from the group consisting of: the anti-TfR1 single domain antibody of the first aspect of the present invention, the anti-TfR1 antibody of the second aspect of the present invention, the chimeric antigen receptor of the third aspect of the present invention, the multispecific antibody of the fourth aspect of the present invention, the recombinant protein of the fifth aspect of the present invention, and combinations thereof.

In another preferred embodiment, the polynucleotide is RNA, DNA, or cDNA.

In a seventh aspect of the present invention, there is provided an expression vector, wherein the expression vector comprises the polynucleotide of the sixth aspect of the present invention.

In another preferred embodiment, the expression vector is selected from the group consisting of: DNA, RNA, viral vectors, plasmids, transposons, other gene transfer systems, and combinations thereof. Preferably, the expression vector includes viral vectors, such as lentivirus, adenovirus, AAV virus, retrovirus, or combinations thereof.

In another preferred embodiment, the expression vector is selected from the group consisting of: pTomo lentiviral vector, plenti, pLVTH, pLJM1, pHCMV, pLBS.CAG, pHR, pLV, etc.

In another preferred embodiment, the expression vector further comprises an element selected from the group consisting of: a promoter, a transcriptional enhancer element WPRE, a long terminal repeat (LTR), etc.

In an eighth aspect of the present invention, there is provided a host cell, wherein the host cell comprises the expression vector of the seventh aspect of the present invention, or has the polynucleotide of the sixth aspect of the present invention integrated into its genome.

In another preferred embodiment, the host cell includes a prokaryotic cell or a eukaryotic cell.

In another preferred embodiment, the host cell is selected from the group consisting of: *Escherichia coli,* yeast cells, and mammalian cells.

In a ninth aspect of the present invention, there is provided an engineered immune cell, wherein the engineered immune cell comprises the expression vector of the seventh aspect of the present invention, or has an exogenous polynucleotide of the sixth aspect of the present invention integrated into its genome, or expresses the chimeric antigen receptor of the third aspect of the present invention.

In another preferred embodiment, the engineered immune cell is selected from the group consisting of:
(i) chimeric antigen receptor αβT cells (CAR-T cells);
(ii) chimeric antigen receptor γδT cells (CAR-T cells);
(iii) chimeric antigen receptor NKT cells (CAR-NKT cells);
(iv) chimeric antigen receptor NK cells (CAR-NK cells).

In another preferred embodiment, the engineered immune cell includes autologous or allogeneic αβT cells, γδT cells, NKT cells, NK cells, or combinations thereof.

In another preferred embodiment, the engineered immune cell is a CAR-T cell.

In a tenth aspect of the present invention, there is provided a method for producing an anti-TfR1 single domain antibody, comprising the steps of:
(a) culturing the host cell of the eighth aspect of the present invention under conditions suitable for producing a single domain antibody, thereby obtaining a culture containing the anti-TfR1 single domain antibody;
(b) isolating and/or recovering the anti-TfR1 single domain antibody from the culture; and
(c) optionally, purifying and/or modifying the anti-TfR1 single domain antibody obtained in step (b).

In an eleventh aspect of the present invention, there is provided an immunoconjugate, wherein the immunoconjugate comprises:
(a) the anti-TfR1 single domain antibody of the first aspect of the present invention, the anti-TfR1 antibody of the second aspect of the present invention, the multispecific antibody of the fourth aspect of the present invention, or the recombinant protein of the fifth aspect of the present invention; and
(b) a conjugated moiety selected from the group consisting of: a detectable label, a drug, a cytokine, a radionuclide, an enzyme, a gold nanoparticle/nanorod, a magnetic nanoparticle, a viral capsid protein, a virus-like particle (VLP), an oligonucleotide, a nucleic acid analog, and a combination thereof.

In another preferred embodiment, moiety (a) is the anti-TfR1 single domain antibody of the first aspect of the present invention, or the anti-TfR1 antibody of the second aspect of the present invention.

In another preferred embodiment, moiety (a) is conjugated to the conjugated moiety via a chemical bond or a linker.

In another preferred embodiment, the radionuclide comprises:
(i) a diagnostic isotope selected from the group consisting of: Tc-99m, Ga-68, F-18, I-123, I-125, I-131, In-111, Ga-67, Cu-64, Zr-89, C-11, Lu-177, Re-188, and combinations thereof; and/or
(ii) a therapeutic isotope selected from the group consisting of: Lu-177, Y-90, Ac-225, As-211, Bi-212, Bi-213, Cs-137, Cr-51, Co-60, Dy-165, Er-169, Fm-255, Au-198, Ho-166, I-125, I-131, Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133, Yb-169, Yb-177, and combinations thereof.

In another preferred embodiment, the conjugated moiety is a drug or a toxin.

In another preferred embodiment, the drug is a drug for targeted therapy of a TfR1 high-expression disease.

In another preferred embodiment, the conjugated moiety is an oligonucleotide.

In another preferred embodiment, the oligonucleotide is capable of regulating gene expression.

In another preferred embodiment, the oligonucleotide is selected from the group consisting of: antisense oligonucleotide (ASO), small interfering RNA (siRNA), microRNA (miRNA), small activating RNA (saRNA), aptamer, ribozyme, DNAzyme, transcription factor decoy, antigene, CpG oligonucleotide, and combinations thereof.

In another preferred embodiment, the nucleic acid analog is selected from the group consisting of: L-nucleic acid, peptide nucleic acid (PNA), morpholino and locked nucleic acid (LNA), phosphorothioate-modified nucleic acid, 2'-fluoro-modified nucleic acid, 5-hydroxymethylcytosine nucleic acid, bridged nucleic acid (BNA), glycol nucleic acid (GNA), threose nucleic acid (TNA), phosphorodiamidate morpholino oligonucleotide (PMO), and combinations thereof.

In another preferred embodiment, the nucleic acid analog is a phosphorodiamidate morpholino oligonucleotide (PMO).

In another preferred embodiment, the TfR1 high-expression disease is selected from the group consisting of: myotonic diseases (e.g., myotonic dystrophy), genetic diseases (e.g., hereditary degenerative diseases, hereditary muscular atrophy diseases), musculoskeletal system diseases, muscular atrophy diseases (e.g., muscular atrophy, Duchenne muscular dystrophy), neuromuscular diseases, myopathies, neurological diseases (e.g., neurodegenerative diseases (e.g., Alzheimer's disease)), muscular dystrophies, iron metabolism disorders, β-thalassemia, iron deficiency anemia, mucopolysaccharidosis type I, mucopolysaccharidosis type II, polycythemia vera, solid tumors (e.g., head and neck cancer, esophageal cancer, brain tumors, nasopharyngeal carcinoma, prostate cancer, breast cancer, ovarian cancer, liver cancer, leukemia, colon cancer, lung cancer), non-solid tumors (e.g., leukemia (e.g., acute lymphoblastic leukemia, acute myeloid leukemia), diffuse large B-cell lymphoma), and combinations thereof.

In another preferred embodiment, the TfR1 high-expression disease is Duchenne muscular dystrophy (DMD).

In another preferred embodiment, the drug is a cytotoxic drug.

In another preferred embodiment, the cytotoxic drug is selected from the group consisting of: anti-microtubule agents, DNA minor groove binders, DNA replication inhibitors, alkylating agents, antibiotics, folate antagonists, antimetabolites, chemosensitizers, topoisomerase inhibitors, vinca alkaloids, and combinations thereof.

Examples of particularly useful classes of cytotoxic drugs include, for example, DNA minor groove binders, DNA alkylating agents, and tubulin inhibitors. Typical cytotoxic drugs include, for example, auristatins, camptothecins, duocarmycins, etoposides, maytansines and maytansinoids (e.g., DM1 and DM4), taxanes, benzodiazepines or benzodiazepine-containing drugs (e.g., pyrrolo[1,4]benzodiazepines (PBDs), indolinobenzodiazepines, and oxazolidinobenzodiazepines), vinca alkaloids, or combinations thereof.

In another preferred embodiment, the toxin is selected from the group consisting of: auristatins (e.g., auristatin E, auristatin F, MMAE, and MMAF), chlortetracycline, maytansinoids, ricin, ricin A-chain, combretastatin, duocarmycin, dolastatin, doxorubicin, daunorubicin, paclitaxel, cisplatin, cc1065, ethidium bromide, mitomycin, etoposide, tenoposide, vincristine, vinblastine, colchicine, dihydroxyanthraquinone, actinomycin, diphtheria toxin, Pseudomonas exotoxin (PE) A, PE40, abrin, abrin A chain, modeccin A chain, α-sarcin, gelonin, mitogellin, restrictocin, phenomycin, enomycin, curicin, crotin, calicheamicin, *Sapaonaria officinalis* inhibitor, glucocorticoids, and combinations thereof.

In another preferred embodiment, the conjugated moiety is a detectable label.

In another preferred embodiment, the conjugated moiety is selected from the group consisting of: fluorescent or luminescent labels, radioactive labels, MRI (Magnetic Resonance Imaging) or CT (Computed Tomography) contrast agents, or enzymes capable of producing a detectable product, radionuclides, biotoxins, cytokines (e.g., IL-2, etc.), antibodies, antibody Fc fragments, antibody scFv fragments, gold nanoparticles/nanorods, viral particles, liposomes, magnetic nanoparticles, prodrug-activating enzymes (e.g., DT-diaphorase (DTD) or biphenyl hydrolase-like protein (BPHL)) or any form of nanoparticles.

In another preferred embodiment, the immunoconjugate comprises multivalent (e.g., bivalent) VHH chains of the anti-TfR1 single domain antibody of the first aspect of the present invention.

In another preferred embodiment, the word "multivalent" means that the amino acid sequence of the immunoconjugate contains multiple repeated copies of the same or different VHH chains of the anti-TfR1 single domain antibody of the first aspect of the present invention.

In a twelfth aspect of the present invention, there is provided a use of an active ingredient selected from the group consisting of: the anti-TfR1 single domain antibody of the first aspect of the present invention, the anti-TfR1 antibody of the second aspect of the present invention, the chimeric antigen receptor of the third aspect of the present invention, the multispecific antibody of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, and combinations thereof, in the manufacture of:
(a) a medicament for preventing and/or treating a TfR1 high-expression disease;
(b) a reagent for detecting a TfR1 high-expression disease.

In another preferred embodiment, the reagent is a diagnostic reagent, preferably the diagnostic reagent is a test strip or a test plate.

In another preferred embodiment, the diagnostic reagent is used for: detecting TfR1 protein or a fragment thereof in a sample.

In another preferred embodiment, the TfR1 high-expression disease is selected from the group consisting of: myotonic diseases (e.g., myotonic dystrophy), genetic diseases (e.g., hereditary degenerative diseases, hereditary muscular atrophy diseases), musculoskeletal system diseases, muscular atrophy diseases (e.g., muscular atrophy, Duchenne muscular dystrophy), neuromuscular diseases, myopathies, neurological diseases (e.g., neurodegenerative diseases (e.g., Alzheimer's disease)), muscular dystrophies, iron metabolism disorders, β-thalassemia, iron deficiency anemia, mucopolysaccharidosis type I, mucopolysaccharidosis type II, polycythemia vera, solid tumors (e.g., head and neck cancer, esophageal cancer, brain tumors, nasopharyngeal carcinoma, prostate cancer, breast cancer, ovarian cancer, liver cancer, leukemia, colon cancer, lung cancer), non-solid tumors (e.g., leukemia (e.g., acute lymphoblastic leukemia, acute myeloid leukemia), diffuse large B-cell lymphoma), and combinations thereof.

In another preferred embodiment, the TfR1 high-expression disease is Duchenne muscular dystrophy (DMD).

In a thirteenth aspect of the present invention, there is provided a method for detecting TfR1 protein or a fragment thereof in a sample *in vitro,* comprising the steps of:
(1) contacting the sample *in vitro* with the anti-TfR1 single domain antibody of the first aspect of the present invention, the anti-TfR1 antibody of the second aspect of the present invention, the chimeric antigen receptor of the third aspect of the present invention, the multispecific antibody of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, or combinations thereof;
(2) detecting whether an antigen-antibody complex is formed, wherein the formation of the complex indicates the presence of TfR1 protein or a fragment thereof in the sample.

In another preferred embodiment, the detection includes diagnostic or non-diagnostic detection.

In a fourteenth aspect of the present invention, there is provided a pharmaceutical composition, wherein the pharmaceutical composition comprises:
(i) the anti-TfR1 single domain antibody of the first aspect of the present invention, the anti-TfR1 antibody of the second aspect of the present invention, the chimeric antigen receptor of the third aspect of the present invention, the multispecific antibody of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, or combinations thereof, as an active ingredient; and
(ii) a pharmaceutically acceptable carrier, diluent, or excipient.

In another preferred embodiment, the dosage form of the pharmaceutical composition is selected from the group consisting of: an injection, and a lyophilized powder.

In another preferred embodiment, the pharmaceutical composition comprises 0.01% to 99.99% of the anti-TfR1 single domain antibody of the first aspect of the present invention, the anti-TfR1 antibody of the second aspect of the present invention, the chimeric antigen receptor of the third aspect of the present invention, the multispecific antibody of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, or combinations thereof, and 0.01% to 99.99% of a pharmaceutically acceptable carrier, wherein the percentage is by mass of the pharmaceutical composition.

In another preferred embodiment, the concentration of the engineered immune cells in the active ingredient is 1×10³ to 1×10⁸ cells/mL, preferably 1×10⁴ to 1×10⁷ cells/mL.

In a fifteenth aspect of the present invention, there is provided a kit, wherein the kit comprises:
(1) a first container containing the anti-TfR1 single domain antibody of the first aspect of the present invention, the anti-TfR1 antibody of the second aspect of the present invention, the chimeric antigen receptor of the third aspect of the present invention, the multispecific antibody of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, or combinations thereof; and/or
(2) a second container containing a secondary antibody against the contents of the first container;
or,
the kit comprises a test strip comprising: a substrate (support plate) and a test strip, wherein the test strip comprises the anti-TfR1 single domain antibody of the first aspect of the present invention, the anti-TfR1 antibody of the second aspect of the present invention, the multispecific antibody of the fourth aspect of the present invention, the recombinant protein of the fifth aspect of the present invention, the immunoconjugate of the eleventh aspect of the present invention, or combinations thereof.

In another preferred embodiment, the kit further comprises an instruction manual, wherein according to the instruction manual, the kit is used for non-invasively detecting TfR1 expression in a subject to be tested.

In another preferred embodiment, the kit is used for detecting a TfR1 high-expression disease.

In another preferred embodiment, the TfR1 high-expression disease is selected from the group consisting of: myotonic diseases (e.g., myotonic dystrophy), genetic diseases (e.g., hereditary degenerative diseases, hereditary muscular atrophy diseases), musculoskeletal system diseases, muscular atrophy diseases (e.g., muscular atrophy, Duchenne muscular dystrophy), neuromuscular diseases, myopathies, neurological diseases (e.g., neurodegenerative diseases (e.g., Alzheimer's disease)), muscular dystrophies, iron metabolism disorders, β-thalassemia, iron deficiency anemia, mucopolysaccharidosis type I, mucopolysaccharidosis type II, polycythemia vera, solid tumors (e.g., head and neck cancer, esophageal cancer, brain tumors, nasopharyngeal carcinoma, prostate cancer, breast cancer, ovarian cancer, liver cancer, leukemia, colon cancer, lung cancer), non-solid tumors (e.g., leukemia (e.g., acute lymphoblastic leukemia, acute myeloid leukemia), diffuse large B-cell lymphoma), and combinations thereof.

In another preferred embodiment, the TfR1 high-expression disease is Duchenne muscular dystrophy (DMD).

In a sixteenth aspect of the present invention, there is provided a method for preventing and/or treating a TfR1 high-expression disease, comprising: administering to a subject in need thereof the anti-TfR1 single domain antibody of the first aspect of the present invention, the anti-TfR1 antibody of the second aspect of the present invention, the chimeric antigen receptor of the third aspect of the present invention, the multispecific antibody of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, the pharmaceutical composition of the fourteenth aspect of the present invention, or combinations thereof.

In another preferred embodiment, the subject comprises a mammal, such as a human.

In another preferred embodiment, the TfR1 high-expression disease is selected from the group consisting of: myotonic diseases (e.g., myotonic dystrophy), genetic diseases (e.g., hereditary degenerative diseases, hereditary muscular atrophy diseases), musculoskeletal system diseases, muscular atrophy diseases (e.g., muscular atrophy, Duchenne muscular dystrophy), neuromuscular diseases, myopathies, neurological diseases (e.g., neurodegenerative diseases (e.g., Alzheimer's disease)), muscular dystrophies, iron metabolism disorders, β-thalassemia, iron deficiency anemia, mucopolysaccharidosis type I, mucopolysaccharidosis type II, polycythemia vera, solid tumors (e.g., head and neck cancer, esophageal cancer, brain tumors, nasopharyngeal carcinoma, prostate cancer, breast cancer, ovarian cancer, liver cancer, leukemia, colon cancer, lung cancer), non-solid tumors (e.g., leukemia (e.g., acute lymphoblastic leukemia, acute myeloid leukemia), diffuse large B-cell lymphoma), and combinations thereof.

In another preferred embodiment, the TfR1 high-expression disease is Duchenne muscular dystrophy (DMD).

In another preferred embodiment, the CAR immune cells contained in the engineered immune cells or the pharmaceutical composition are cells derived from the subject (autologous cells).

In another preferred embodiment, the CAR immune cells contained in the engineered immune cells or the pharmaceutical composition are cells derived from a healthy individual (allogeneic cells).

In another preferred embodiment, the method may be used in combination with other therapeutic methods.

In another preferred embodiment, the other therapeutic methods include chemotherapy, radiotherapy, targeted therapy, etc.

In a seventeenth aspect of the present invention, there is provided a method for diagnosing a TfR1 high-expression disease, comprising the steps of:
(i) obtaining a sample from a subject for diagnosis, and contacting the sample with the anti-TfR1 single domain antibody of the first aspect of the present invention, the anti-TfR1 antibody of the second aspect of the present invention, the chimeric antigen receptor of the third aspect of the present invention, the multispecific antibody of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, or combinations thereof; and
(ii) detecting whether an antigen-antibody complex is formed, wherein the formation of the complex indicates that the subject is a patient diagnosed with a TfR1 high-expression disease.

In another preferred embodiment, the sample is a blood sample, a throat swab sample, or a sample from other tissues or organs.

In another preferred embodiment, the TfR1 high-expression disease is selected from the group consisting of: myotonic diseases (e.g., myotonic dystrophy), genetic diseases (e.g., hereditary degenerative diseases, hereditary muscular atrophy diseases), musculoskeletal system diseases, muscular atrophy diseases (e.g., muscular atrophy, Duchenne muscular dystrophy), neuromuscular diseases, myopathies, neurological diseases (e.g., neurodegenerative diseases (e.g., Alzheimer's disease)), muscular dystrophies, iron metabolism disorders, β-thalassemia, iron deficiency anemia, mucopolysaccharidosis type I, mucopolysaccharidosis type II, polycythemia vera, solid tumors (e.g., head and neck cancer, esophageal cancer, brain tumors, nasopharyngeal carcinoma, prostate cancer, breast cancer, ovarian cancer, liver cancer, leukemia, colon cancer, lung cancer), non-solid tumors (e.g., leukemia (e.g., acute lymphoblastic leukemia, acute myeloid leukemia), diffuse large B-cell lymphoma), and combinations thereof.

In another preferred embodiment, the TfR1 high-expression disease is Duchenne muscular dystrophy (DMD).

In an eighteenth aspect of the present invention, there is provided a method for preparing a recombinant polypeptide, wherein the recombinant polypeptide is the anti-TfR1 single domain antibody of the first aspect of the present invention, the anti-TfR1 antibody of the second aspect of the present invention, the chimeric antigen receptor of the third aspect of the present invention, the multispecific antibody of the fourth aspect of the present invention, the recombinant protein of the fifth aspect of the present invention, or combinations thereof, and the method comprises:
(a) culturing the host cell of the eighth aspect of the present invention under conditions suitable for expression; and
(b) isolating the recombinant polypeptide from the culture.

It should be understood that within the scope of the present invention, the above-mentioned technical features of the present invention and the technical features specifically described in the following (such as the examples) can be combined with each other to form a new or preferred technical solution, which is not redundantly repeated one by one herein due to space limitation.

### DESCRIPTION OF THE DRAWINGS

Figure 1 shows the protein sequence alignment results of TfR1 antigens from human, rhesus monkey, and murine species.
Figure 2 shows the construction of the yeast library for anti-TfR1 single-domain antibodies; panel a: counting plate of library transformants; panel b: detection of antibody sequence insertion rate in the library; panel c: phylogenetic tree analysis of amino acid sequence diversity in the library.
Figure 3 shows SDS-PAGE purity identification of the anti-TfR1 single domain antibody during expression and purification process; left: supernatant after 48 hours of induced expression; right: nickel ion affinity chromatography purified sample after 72 hours of induced expression.
Figure 4 shows ELISA detection of the binding ability of anti-TfR1 single domain antibodies to TfR1 antigens from three species; upper left: binding activity to human TfR1 antigen; upper right: binding activity to monkey TfR1 antigen; lower left: binding activity to murine TfR1 antigen.
Figure 5 shows ELISA detection of the binding ability of anti-TfR1 single domain antibodies to human TfR1 antigen.
Figure 6 shows ELISA detection of the binding ability of anti-TfR1 single domain antibodies to monkey and murine TfR1 antigens; left: binding activity to monkey TfR1 antigen; right: binding activity to murine TfR1 antigen.
Figure 7 shows flow cytometric detection of the binding ability of anti-TfR1 single domain antibodies to CHO-K1 cells highly expressing hTfR1 or hTfR2; left: binding activity to hTfR1 CHO-K1 cells; right: binding activity to hTfR2 CHO-K1 cells.
Figure 8 shows flow cytometric detection of the binding ability of anti-TfR1 single domain antibodies to hTfR1 CHO-K1 cells.
Figure 9 shows ELISA detection of the binding ability of anti-TfR1 single domain antibodies to hTfR1 pre-bound with transferrin.
Figure 10 shows the alignment results of candidate antibody TfR1-95 with human Germline sequences.
Figure 11 shows ELISA detection of the binding ability of humanized anti-TfR1 VHH-Fc to human TfR1 antigen.
Figure 12 shows ELISA detection of the binding ability of humanized anti-TfR1 VHH-Fc to monkey TfR1 antigen.
Figure 13 shows flow cytometric detection of the binding ability of humanized anti-TfR1 VHH-Fc to hTfR1 CHO-K1 cells.
Figure 14 shows the identification of TfR1 expression on the surface of SK-OV-3 tumor cells.
Figure 15 shows ELISA detection of the binding ability of humanized anti-TfR1 VHH-Fc to hTfR1 pre-bound with recombinant human HFE protein.
Figure 16 shows the chromatogram and SDS-PAGE verification of the anti-TfR1 single domain antibody-PMO conjugate.
Figure 17 shows the agarose gel electrophoresis analysis results of wild-type DMD products and Exon 23-skipped DMD products in groups G1, G2, and G3, and the corresponding Exon 23 skipping rates.
Figure 18 shows the verification results of sequencing analysis of the exon-skipped bands.

### DETAILED DESCRIPTION

Through extensive and in-depth research and massive screening, the inventors unexpectedly obtained, for the first time, anti-TfR1 single-domain antibodies with high affinity and high specificity. Specifically, in the present invention, healthy adult alpacas were immunized with human TfR1 antigen. Upon obtaining a high-quality yeast library of 10⁸, 44 single-domain antibodies positive for binding to both human and monkey TfR1 antigens were identified using flow cytometry, and humanized antibodies were further obtained. The binding epitopes of the anti-TfR1 single domain antibodies of the present invention may be the same as or different from that of transferrin, and they exhibit a non-competitive relationship with the HFE protein. Therefore, they can be used for the treatment and/or diagnosis of different types of related diseases, and drugs or formulations prepared based on said antibodies are less affected by the *in vivo* environment. Furthermore, compared to the control hIgG1 antibody, the anti-TfR1 single-domain antibodies of the present invention exhibit higher internalization activity on tumor cells, enabling the preparation of drugs with higher efficacy. On this basis, the present invention has been completed.

### TERMS

In order to facilitate the understanding of the present disclosure, certain terms are first defined. As used in this application, unless otherwise expressly provided herein, each of the following terms shall have the meaning set forth below.

The term "about" may refer to a value or composition within an acceptable error range for a particular value or composition as determined by one of ordinary skill in the art, which will depend in part on how the value or composition is measured or determined.

The term "administration" refers to physically introducing a product of the invention into a subject using any of the various methods and delivery systems known to those skilled in the art, including intravenous, intratumoral, intramuscular, subcutaneous, intraperitoneal, spinal or other parenteral routes of administration, for example by injection or infusion.

The names and corresponding sequence identification numbers of the TfR1-targeting single domain antibodies of the present invention are shown in Table A below.

**Table A**

| Antibody Name | CDR1 | CDR2 | CDR3 | FR1 | FR2 | FR3 | FR4 | VHH Chain Amino Acid SEQ ID NO |
|---|---|---|---|---|---|---|---|---|
| TfR1-3 | 57 | 76 | 77 | 120 | 121 | 122 | 123 | 1 |
| | | | | (QVQLV ESGGGL VQPGGS LRLSCA AS) | (MTW VRQA PGKE LEWV SS) | (DYAGSVK GRFTISRDN AKNTLYLQ MNSLKPED TAVYYC) | (KGQ GTQV IVSS) | |
| TfR1-6 | 81 | 79 | 80 | 124 | 125 | 126 | 127 | 2 |
| | | | | (QVQLV ESGGGS VQTGGS LKLSCA AS) | (MSW VRQA PGKG LEWV SG) | (RYADSVK GRFTISRDI AKNTMYLQ MNDLRSED TAVYYC) | (WGQ GTQV TVSS) | |
| TfR1-12 | 96 | 70 | 83 | 128 | 129 | 130 | 127 | 3 |
| | | | | (EVQLVE SGGGLV QAGGSL RLSCAA S) | (MAW YRRP PGKQ HELL AV) | (KYVGSAR GRFTVSRD NAKSTLYL QMTSLKPE DTAVYYC) | (WGQ GTQV TVSS) | |
| TfR1-13 | 48 | 49 | 86 | 131 | 125 | 132 | 127 | 4 |
| | | | | (EVQLVE SGGDLV QPGGSL RLSCAA S) | (MSW VRQA PGKG LEWV SG) | (SYADSVK GRFTISRDN AKNSVDLQ MTSLKPED TAIYYC) | (WGQ GTQV TVSS) | |
| TfR1-16 | 45 | 46 | 47 | 133 | 134 | 135 | 136 | 5 |
| | | | | (QLQLVE SGGGLV QPGGSL | (MSW LRQA PGKG | (DYADSVK GRFTISRDN AKNTLYLQ | (RGQ GTQV TVSS) | |
| | | | | RLSCAA S) | LEWV SS) | MNSLKPED TAVYYC) | | |
| TfR1-21 | 54 | 61 | 53 | 137 | 138 | 139 | 140 | 6 |
| | | | | (EVQLVE SGGGVV QTGGSL RLSCAA S) | (MSW LRQA PGKG LEWV SA) | (SYADSVK GRFTIARDN AKNTLYLQ MNTLKPED TAVYYC) | (KGQ GTQV TVSS) | |
| TfR1-23 | 54 | 61 | 56 | 141 | 138 | 139 | 140 | 7 |
| | | | | (EVQLVE SGGGLV QPGGSL RLSCAA S) | (MSW LRQA PGKG LEWV SA) | (SYADSVK GRFTIARDN AKNTLYLQ MNTLKPED TAVYYC) | (KGQ GTQV TVSS) | |
| TfR1-27 | 93 | 82 | 89 | 142 | 143 | 144 | 127 | 8 |
| | | | | (QVQLV ESGGGL VQAGGS LRLSCA AS) | (MGW YRQA SGKE REFV AN) | (NYADSVK DRFTISRDN RKNTVYLQ MDSLTLED TAVYYC) | (WGQ GTQV TVSS) | |
| TfR1-37 | 75 | 58 | 68 | 145 | 146 | 147 | 148 | 9 |
| | | | | (QLQLVE SGGGLV QSGGSL RLSCTV S) | (IKWF RQAP GKER EGVS C) | (YYSDSVK GRFTISRDN AKNTVYLQ MNSLKPED TAVYYC) | (WGQ GTLV TVSS) | |
| TfR1-41 | 48 | 49 | 92 | 141 | 149 | 150 | 127 | 10 |
| | | | | (EVQLVE SGGGLV QPGGSL RLSCAA S) | (MSW VRQA PGKG LEWV ST) | (SYADSVK GRFTISRDN AKNTLYLQ MNSLKPED TAVYYC) | (WGQ GTQV TVSS) | |
| TfR1-45 | 69 | 64 | 74 | 128 | 151 | 152 | 153 | 11 |
| | | | | (EVQLVE SGGGLV QAGGSL RLSCAA S) | (ISWF RQAP GKER EGVC S) | (VYADSVK GRFTISRDS AKNTVYLQ MNSLKPED TAVYYC) | (WGK GTQV TVSS) | |
| TfR1-72 | 111 | 85 | 62 | 154 | 155 | 156 | 127 | 12 |
| | | | | (EAEAQL QVVESG GGLVQP GGSLSLS CAVS) | (IGWF RQAP GKQR ELVA S) | (NYENSVK GRFTISRDS ARNTAYLQ MNDLKPED TAIYYC) | (WGQ GTQV TVSS) | |
| TfR1-74 | 75 | 58 | 95 | 157 | 146 | 147 | 148 | 13 |
| | | | | (EVQLVE SGGGLV QSGGSL RLSCTV S) | (IKWF RQAP GKER EGVS C) | (YYSDSVK GRFTISRDN AKNTVYLQ MNSLKPED TAVYYC) | (WGQ GTLV TVSS) | |
| TfR1-95 | 51 | 73 | 98 | 133 | 134 | 135 | 136 | 14 |
| | | | | (QLQLVE SGGGLV QPGGSL RLSCAA S) | (MSW LRQA PGKG LEWV SS) | (DYADSVK GRFTISRDN AKNTLYLQ MNSLKPED TAVYYC) | (RGQ GTQV TVSS) | |
| TfR1-105 | 69 | 64 | 74 | 158 | 151 | 152 | 159 | 15 |
| | | | | (EVQVV ESGGGL VHPGGS LRLSCA AS) | (ISWF RQAP GKER EGVC S) | (VYADSVK GRFTISRDS AKNTVYLQ MNSLKPED TAVYYC) | (WGK GTLV TVSS) | |
| TfR1-109 | 45 | 46 | 47 | 141 | 134 | 160 | 136 | 16 |
| | | | | (EVQLVE SGGGLV QPGGSL RLSCAA S) | (MSW LRQA PGKG LEWV SS) | (DYADSVK GRYTISRDN AKNTLYLQ MSSLKPED TAVYYC) | (RGQ GTQV TVSS) | |
| TfR1-111 | 45 | 52 | 47 | 161 | 134 | 135 | 136 | 17 |
| | | | | (QVQLV ESGGGS VQPGGS LRLSCT AS) | (MSW LRQA PGKG LEWV SS) | (DYADSVK GRFTISRDN AKNTLYLQ MNSLKPED TAVYYC) | (RGQ GTQV TVSS) | |
| TfR1-114 | 45 | 49 | 50 | 120 | 162 | 150 | 136 | 18 |
| | | | | (QVQLV ESGGGL VQPGGS LRLSCA AS) | (MSW VRQA PGKG LEWV SA) | (SYADSVK GRFTISRDN AKNTLYLQ MNSLKPED TAVYYC) | (RGQ GTQV TVSS) | |
| TfR1-120 | 45 | 46 | 47 | 163 | 134 | 135 | 136 | 19 |
| | | | | (EVQVV ESGGGL VQPGGS LRLSCA AS) | (MSW LRQA PGKG LEWV SS) | (DYADSVK GRFTISRDN AKNTLYLQ MNSLKPED TAVYYC) | (RGQ GTQV TVSS) | |
| TfR1-121 | 54 | 61 | 53 | 141 | 138 | 139 | 164 | 20 |
| | | | | (EVQLVE SGGGLV QPGGSL RLSCAA S) | (MSW LRQA PGKG LEWV SA) | (SYADSVK GRFTIARDN AKNTLYLQ MNTLKPED TAVYYC) | (KGQ GTQV TVSG) | |
| TfR1-131 | 75 | 58 | 68 | 157 | 146 | 147 | 148 | 21 |
| | | | | (EVQLVE SGGGLV QSGGSL RLSCTV S) | (IKWF RQAP GKER EGVS C) | (YYSDSVK GRFTISRDN AKNTVYLQ MNSLKPED TAVYYC) | (WGQ GTLV TVSS) | |
| TfR1-132 | 63 | 55 | 65 | 142 | 165 | 166 | 127 | 22 |
| | | | | (QVQLV ESGGGL VQAGGS LRLSCA AS) | (LVW FRQA PGKE REGV SC) | (YYADSVK GRFTISRDN AKNTVYLQ MNSLKPED TAVYYC) | (WGQ GTQV TVSS) | |
| TfR1-134 | 60 | 67 | 71 | 141 | 167 | 168 | 136 | 23 |
| | | | | (EVQLVE SGGGLV QPGGSL RLSCAA S) | (MTW ARQA PGKG LEWV SG) | (QYADSVK GRFTISRDN AKNTLYLQ MNSLKPED TAVYYC) | (RGQ GTQV TVSS) | |
| TfR1-135 | 108 | 88 | 101 | 169 | 170 | 171 | 172 | 24 |
| | | | | (EVQVV ESGGGL VQAGDS LRLSCA | (MDW YRQA PGKE REFV | (HYADSVE GRFTISRSN GKNTVYLQ MNSLKTED | (WGR GTQV TVSS) | |
| | | | | VS) | AR) | TAIYYC) | | |
| TfR1-401 | 87 | 91 | 62 | 173 | 174 | 175 | 127 | 25 |
| | | | | (QVQLV ESGGGL VQPGAS LRLSCT AS) | (MGW YRQA PGKK REFV AA) | (HYTDSVK GRFTIARDN AKSTVYLQ MLSLKPDD TAIYYC) | (WGQ GTQV TVSS) | |
| TfR1-415 | 90 | 94 | 104 | 176 | 177 | 178 | 179 | 26 |
| | | | | (EVQLVE SGGGTA QPGGSL RLSCAA S) | (MGW YRQA PGKE REFV AG) | (SYANSVK GRFTISRDN AKKTLYLQ MNSLKPED TAIYYC) | (WGP GTQV TVSS) | |
| TfR1-429 | 102 | 97 | 59 | 180 | 181 | 182 | 127 | 27 |
| | | | | (QVQLV ESGGGL VQVGGS LRLSCV AS) | (MGW YRQA PGKQ RELV AS) | (NYADSVK GRFTISRDG TKNMVYLQ MNNLKPED TAVYYC) | (WGQ GTQV TVSS) | |
| TfR1-432 | 45 | 100 | 50 | 133 | 134 | 183 | 136 | 28 |
| | | | | (QLQLVE SGGGLV QPGGSL RLSCAA S) | (MSW LRQA PGKG LEWV SS) | (DAANSVK GRFTISRDN AKNTLYLQ MNSLKPED TAVYYC) | (RGQ GTQV TVSS) | |
| TfR1-434 | 45 | 49 | 50 | 163 | 162 | 150 | 136 | 29 |
| | | | | (EVQVV ESGGGL VQPGGS LRLSCA AS) | (MSW VRQA PGKG LEWV SA) | (SYADSVK GRFTISRDN AKNTLYLQ MNSLKPED TAVYYC) | (RGQ GTQV TVSS) | |
| TfR1-436 | 84 | 103 | 107 | 184 | 185 | 186 | 127 | 30 |
| | | | | (QVQLV ESGGGL VQPGGS LRLSCT AS) | (MGW YRQA PGKE RELV AA) | (HYADSVK GRFTISRDT AKKTLYLQ MNSLRSDD TAVYYC) | (WGQ GTQV TVSS) | |
| TfR1-446 | 75 | 58 | 68 | 187 | 146 | 147 | 148 | 31 |
| | | | | (EVQVV ESGGGL VQAGGS LRLSCT VS) | (IKWF RQAP GKER EGVS C) | (YYSDSVK GRFTISRDN AKNTVYLQ MNSLKPED TAVYYC) | (WGQ GTLV TVSS) | |
| TfR1-449 | 69 | 64 | 74 | 188 | 151 | 152 | 159 | 32 |
| | | | | (EVQLVE SGGGLV HPGGSL RLSCAA S) | (ISWF RQAP GKER EGVC S) | (VYADSVK GRFTISRDS AKNTVYLQ MNSLKPED TAVYYC) | (WGK GTLV TVSS) | |
| TfR1-456 | 99 | 106 | 110 | 189 | 190 | 191 | 127 | 33 |
| | | | | (QLQLVE SGGGLV QAGGSL RLSCAV S) | (MAW YRQA PGKQ RELV AH) | (TYTDSVK GRFTISRDS TKNTVVLQ MNSLKPED TAVYFC) | (WGQ GTQV TVSS) | |
| TfR1-528 | 54 | 61 | 53 | 120 | 192 | 193 | 140 | 34 |
| | | | | (QVQLV ESGGGL VQPGGS LRLSCA AS) | (MSW VRQA PGKA LEWV SA) | (SYADSVK GRFTIARDN AKNTLYLQ MNSLKPED TAVYYC) | (KGQ GTQV TVSS) | |
| TfR1-546 | 105 | 109 | 113 | 194 | 195 | 196 | 127 | 35 |
| | | | | (QVQLV ESGGGL VQAGGS LRLSCT AS) | (MGW YRQA PDKQ RELV AS) | (KYSDSVK GRFTISRDN AKNTVYLK MDSLKPED TAIYYC) | (WGQ GTQV TVSS) | |
| TfR1-547 | 78 | 58 | 68 | 197 | 198 | 147 | 148 | 36 |
| | | | | (QLQLVE SGGGLV QPGDSL RLSCTA S) | (IGWF RQAP GKER EGVS C) | (YYSDSVK GRFTISRDN AKNTVYLQ MNSLKPED TAVYYC) | (WGQ GTLV TVSS) | |
| TfR1-554 | 54 | 61 | 56 | 163 | 138 | 199 | 140 | 37 |
| | | | | (EVQVV ESGGGL VQPGGS LRLSCA AS) | (MSW LRQA PGKG LEWV SA) | (SYADSVK GRFTIARDN AEDTVYLQ MNTLKPED TAVYYC) | (KGQ GTQV TVSS) | |
| TfR1-572 | 96 | 112 | 115 | 200 | 201 | 202 | 127 | 38 |
| | | | | (QLQLV ESGGGL VQAGGS LRLSCA AS) | (MAW YRQP PGKQ RELL AV) | (KYVGSAK GRFTVSRD NARNTLYL QMNSLKPE DTAVYYC) | (WGQ GTQV TVSS) | |
| TfR1-584 | 48 | 114 | 117 | 120 | 203 | 204 | 127 | 39 |
| | | | | (QVQLV ESGGGL VQPGGS LRLSCA AS) | (MSW VRQA PGKG LEWV SS) | (AYSDSVK GRFTISRDA AKNTVYLQ MNSLKPED TAVYYC) | (WGQ GTQV TVSS) | |
| TfR1-589 | 72 | 64 | 74 | 205 | 151 | 152 | 159 | 40 |
| | | | | (QLQLVE SGGGLV QPGESL RLSCVV S) | (ISWF RQAP GKER EGVC S) | (VYADSVK GRFTISRDS AKNTVYLQ MNSLKPED TAVYYC) | (WGK GTLV TVSS) | |
| TfR1-595 | 51 | 46 | 118 | 120 | 134 | 135 | 136 | 41 |
| | | | | (QVQLV ESGGGL VQPGGS LRLSCA AS) | (MSW LRQA PGKG LEWV SS) | (DYADSVK GRFTISRDN AKNTLYLQ MNSLKPED TAVYYC) | (RGQ GTQV TVSS) | |
| TfR1-597 | 66 | 116 | 119 | 206 | 207 | 208 | 127 | 42 |
| | | | | (QVQLV ESGGTL GQPGGS LRLSCA AS) | (MTW VRQA PGKG LEWV ST) | (RYADSVK GRFTISRDN AKNTLYLQ MNNLTPED TAVYYC) | (WGQ GTQV TVSS) | |
| TfR1-599 | 60 | 67 | 71 | 209 | 167 | 168 | 136 | 43 |
| | | | | (QVQVV ESGGGL VQPGGS LRLSCA | (MTW ARQA PGKG LEWV | (QYADSVK GRFTISRDN AKNTLYLQ MNSLKPED | (RGQ GTQV TVSS) | |
| | | | | AS) | SG) | TAVYYC) | | |
| TfR1-606 | 63 | 55 | 65 | 128 | 165 | 166 | 127 | 44 |
| | | | | (EVQLVE SGGGLV QAGGSL RLSCAA S) | (LVW FRQA PGKE REGV SC) | (YYADSVK GRFTISRDN AKNTVYLQ MNSLKPED TAVYYC) | (WGQ GTQV TVSS) | |
| AS001-N0 37-095-V HH1 | 51 | 73 | 98 | 133 | 134 | 135 | 148 | 210 |
| | | | | (QLQLVE SGGGLV QPGGSL RLSCAA S) | (MSW LRQA PGKG LEWV SS) | (DYADSVK GRFTISRDN AKNTLYLQ MNSLKPED TAVYYC) | (WGQ GTLV TVSS) | |
| AS001-N0 37-095-V HH2 | 51 | 73 | 98 | 133 | 134 | 216 | 127 | 211 |
| | | | | (QLQLVE SGGGLV QPGGSL RLSCAA S) | (MSW LRQA PGKG LEWV SS) | (DYADSVK GRFTISRDN AKNTLYLQ MNSLRAED TAVYYC) | (WGQ GTQV TVSS) | |
| AS001-N0 37-095-V HH3 | 51 | 73 | 98 | 133 | 134 | 216 | 217 | 212 |
| | | | | (QLQLVE SGGGLV QPGGSL RLSCAA S) | (MSW LRQA PGKG LEWV SS) | (DYADSVK GRFTISRDN AKNTLYLQ MNSLRAED TAVYYC) | (RGQ GTLV TVSS) | |
| AS001-N0 37-095-V HH4 | 51 | 73 | 98 | 133 | 134 | 216 | 148 | 213 |
| | | | | (QLQLVE SGGGLV QPGGSL RLSCAA S) | (MSW LRQA PGKG LEWV SS) | (DYADSVK GRFTISRDN AKNTLYLQ MNSLRAED TAVYYC) | (WGQ GTLV TVSS) | |
| AS001-N0 37-095-V HH5 | 51 | 73 | 98 | 141 | 134 | 216 | 217 | 214 |
| | | | | (EVQLVE SGGGLV QPGGSL RLSCAA S) | (MSW LRQA PGKG LEWV SS) | (DYADSVK GRFTISRDN AKNTLYLQ MNSLRAED TAVYYC) | (RGQ GTLV TVSS) | |
| AS001-N0 37-095-V HH6 | 51 | 73 | 98 | 141 | 134 | 216 | 148 | 215 |
| | | | | (EVQLVE SGGGLV QPGGSL RLSCAA S) | (MSW LRQA PGKG LEWV SS) | (DYADSVK GRFTISRDN AKNTLYLQ MNSLRAED TAVYYC) | (WGQ GTLV TVSS) | |

| | | | | | | | | |
|---|---|---|---|---|---|---|---|---|
| Note: The numbers in the table represent SEQ ID NOs, i.e., "1" represents "SEQ ID NO: 1". The sequence numbers for CDR1, CDR2, CDR3, FR1, FR2, FR3, and FR4 shown in the table are the SEQ ID NOs of their respective amino acid sequences. | | | | | | | | |

The above CDRs of the anti-TfR1 single domain antibodies of the present invention are defined according to the IMGT numbering scheme. Those skilled in the art shall understand that when other CDR definition schemes, such as the Kabat scheme, AbM scheme, Chothia scheme, or Contact scheme, are used for definition, the corresponding CDRs also fall within the protection scope of the present invention.

As used herein, the term "antibody" or "immunoglobulin" is a heterotetrameric glycoprotein of about 150,000 Daltons with the same structural characteristics, composed of two identical light chains (L) and two identical heavy chains (H). Each light chain is linked to a heavy chain by a covalent disulfide bond, and the number of disulfide bonds between heavy chains of different immunoglobulin isotypes varies. Each heavy and light chain also has regularly spaced intrachain disulfide bonds. Each heavy chain has a variable region (VH) at one end, followed by multiple constant regions. Each light chain has a variable region (VL) at one end and a constant region at the other; the constant region of the light chain is opposite the first constant region of the heavy chain, and the variable region of the light chain is opposite the variable region of the heavy chain. Special amino acid residues form an interface between the variable regions of the light and heavy chains.

As used herein, the terms "single domain antibody (sdAb)", "VHH", and "nanobody" have the same meaning and may be used interchangeably, referring to a single domain antibody consisting of only one heavy chain variable region, which is cloned from the variable region of an antibody heavy chain, and is the smallest antigen-binding fragment with complete function. Typically, an antibody naturally lacking light chains and the heavy chain constant region 1 (CH1) is first obtained, and then the variable region of the antibody heavy chain is cloned to construct a single domain antibody (VHH) consisting of only one heavy chain variable region.

As used herein, the term "variable" means that certain portions of the variable regions of antibodies differ in sequence, which forms the binding and specificity of various specific antibodies for their specific antigens. However, variability is not evenly distributed throughout the antibody variable regions. It is concentrated in three segments called complementarity determining regions (CDRs) or hypervariable regions in the variable regions of the light and heavy chains. The more conserved portions of the variable regions are called framework regions (FR). The variable regions of native heavy and light chains each contain four FR regions, which are largely in a β-sheet configuration, connected by three CDRs forming loops, and in some cases may form part of the β-sheet structure. The CDRs in each chain are held together in close proximity by the FR regions and, together with the CDRs from the other chain, form the antigen-binding site of the antibody (see Kabat et al., NIH Publ. No. 91-3242, Vol. I, pages 647-669 (1991)). The constant regions are not directly involved in antibody -antigen binding, but they exhibit different effector functions, such as participation in antibody-dependent cellular cytotoxicity.

As known to those skilled in the art, immunoconjugates and fusion expression products include: conjugates formed by binding drugs, toxins, cytokines, radionuclides, enzymes and other diagnostic or therapeutic molecules to the antibodies or fragments thereof of the present invention. The present invention also includes cell surface markers or antigens that bind to the single domain antibodies or fragments thereof directed against TfR1.

As used herein, the terms "hypervariable region", "highly variable region", "complementarity determining region" and "complementarity determining region (CDR)" may be used interchangeably.

In a preferred embodiment of the present invention, the heavy chain variable region of the single domain antibody or antibody comprises three complementarity determining regions CDR1, CDR2, and CDR3.

In a preferred embodiment of the present invention, the heavy chain of the single domain antibody or antibody comprises the above heavy chain variable region and heavy chain constant regions.

In the present invention, the terms "nanobody", "single domain antibody", "single domain antibody of the invention", "nanobody of the invention", "antibody of the invention", "protein of the invention", or "polypeptide of the invention" may be used interchangeably, all referring to a polypeptide that specifically binds to TfR1 protein, for example, a protein or polypeptide having a heavy chain variable region. They may or may not contain an initiating methionine.

The present invention also provides other proteins or fusion expression products having the antibodies of the present invention. Specifically, the present invention includes any protein or protein conjugate and fusion expression product having a heavy chain containing a variable region, provided that the variable region is identical to or has at least 90% identity, preferably at least 95% identity, to the heavy chain variable region of the antibody of the present invention.

Generally, the antigen-binding characteristics of an antibody can be described by three specific regions in the heavy chain variable region, called variable regions (CDRs), which are separated into four framework regions (FRs). The amino acid sequences of the four FRs are relatively conserved and do not directly participate in binding reactions. These CDRs form loop structures, and through the β-sheets formed by the intervening FRs, they are close to each other in spatial structure. The CDRs on the heavy chain and the corresponding CDRs on the light chain constitute the antigen-binding site of the antibody. It can be determined which amino acids constitute the FR or CDR regions by comparing the amino acid sequences of antibodies of the same type.

The heavy chain variable regions of the single domain antibodies or antibodies of the present invention are of particular interest because at least some of them are involved in antigen binding. Therefore, the present invention includes those molecules having antibody heavy chain variable regions with CDRs, provided that the CDRs have more than 90% (preferably more than 95%, most preferably more than 98%) identity with the CDRs identified herein.

The present invention includes not only intact antibodies but also fragments of antibodies with immunological activity or fusion proteins formed by antibodies with other sequences. Accordingly, the present invention also includes fragments, derivatives, and analogs of the antibodies.

As used herein, the terms "fragment", "derivative", and "analog" refer to a polypeptide that substantially retains the same biological function or activity of the antibody of the present invention. The polypeptide fragments, derivatives or analogs of the present invention may be (i) a polypeptide in which one or more conservative or non-conservative amino acid residues (preferably conservative amino acid residues) are substituted, and such substituted amino acid residues may or may not be encoded by the genetic code, or (ii) a polypeptide having a substituent group in one or more amino acid residues, or (iii) a polypeptide formed by fusing a mature polypeptide with another compound (such as a compound that extends the half-life of the polypeptide, e.g., polyethylene glycol), or (iv) a polypeptide formed by fusing an additional amino acid sequence to this polypeptide sequence (such as a leader or secretory sequence or a sequence used to purify this polypeptide or a proprotein sequence, or a fusion protein formed with a 6His tag). According to the teachings herein, these fragments, derivatives and analogs are within the knowledge of those skilled in the art.

The antibodies of the present invention refer to polypeptides that have TfR1 protein binding activity and include the above CDR regions. The term also includes variant forms of the polypeptide that have the same function as the antibody of the present invention and include the above CDR regions. These variant forms include (but are not limited to): deletion, insertion and/or substitution of one or more (usually 1-50, preferably 1-30, more preferably 1-20, most preferably 1-10) amino acids, and addition of one or more (usually within 20, preferably within 10, more preferably within 5) amino acids at the C-terminus and/or N-terminus. For example, in the art, substitution with amino acids of similar or close properties usually does not alter protein function. Similarly, adding one or more amino acids at the C-terminus and/or N-terminus usually does not alter protein function. The term also includes active fragments and active derivatives of the antibodies of the present invention.

Variant forms of the polypeptide include: homologous sequences, conservative variants, allelic variants, natural mutants, induced mutants, proteins encoded by DNA that hybridizes with the DNA encoding the antibody of the present invention under high or low stringency conditions, and polypeptides or proteins obtained using antisera against the antibody of the present invention.

The present invention also provides other polypeptides, such as fusion proteins containing the antibody or fragment thereof. In addition to almost full-length polypeptides, the present invention also includes fragments of the antibodies of the present invention. Typically, the fragment has at least about 50 consecutive amino acids of the antibody of the present invention, preferably at least about 50 consecutive amino acids, more preferably at least about 80 consecutive amino acids, most preferably at least about 100 consecutive amino acids.

In the present invention, "conservative variants of the antibody of the invention" refer to polypeptides formed by replacing at most 10, preferably at most 8, more preferably at most 5, most preferably at most 3 amino acids in the amino acid sequence of the antibody of the present invention with amino acids of similar or analogous properties. These conservative variant polypeptides are preferably produced by amino acid substitutions according to Table B.

**Table B**

| Original Residue | Representative Substitutions | Preferred Substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides polynucleotide molecules encoding the above antibodies or fragments thereof or fusion proteins thereof. The polynucleotides of the present invention can be in DNA form or RNA form. DNA forms include cDNA, genomic DNA, or synthetic DNA. DNA can be single-stranded or double-stranded. DNA can be the coding strand or the non-coding strand.

Polynucleotides encoding the mature polypeptides of the present invention include: coding sequences encoding only the mature polypeptide; coding sequences for the mature polypeptide and various additional coding sequences; coding sequences for the mature polypeptide (and optionally additional coding sequences) and non-coding sequences.

The term "polynucleotide encoding a polypeptide" may include a polynucleotide that includes the coding sequence for this polypeptide, or may also include additional coding and/or non-coding sequences.

The present invention also relates to polynucleotides that hybridize to the above sequences and have at least 50%, preferably at least 70%, more preferably at least 80% identity between the two sequences. The present invention particularly relates to polynucleotides that can hybridize to the polynucleotides described herein under stringent conditions. In the present invention, "stringent conditions" refer to: (1) hybridization and washing at lower ionic strength and higher temperature, such as 0.2×SSC, 0.1% SDS, 60°C; or (2) hybridization with a denaturant, such as 50% (v/v) formamide, 0.1% calf serum/0.1% Ficoll, 42°C, etc.; or (3) hybridization occurs only when the identity between the two sequences is at least 90%, more preferably at least 95%. Moreover, the polypeptide encoded by the hybridizable polynucleotide has the same biological function and activity as the mature polypeptide.

The full-length nucleotide sequence of the antibody of the present invention or a fragment thereof can usually be obtained by PCR amplification, recombinant methods, or synthetic methods. One feasible method is to synthesize the relevant sequences by artificial synthesis, especially when the fragment length is short. Typically, long fragments can be obtained by synthesizing multiple small fragments first and then ligating them. Furthermore, the coding sequence of the heavy chain can be fused with an expression tag (such as 6His) to form a fusion protein.

Once the relevant sequence is obtained, it can be obtained in large quantities by recombinant methods. This is usually done by cloning it into a vector, transforming it into cells, and then isolating the relevant sequence from the propagated host cells by conventional methods. The biomolecules (nucleic acids, proteins, etc.) involved in the present invention include biomolecules in isolated form.

Currently, the DNA sequence encoding the protein (or fragment thereof, or derivative thereof) of the present invention can be completely obtained by chemical synthesis. This DNA sequence can then be introduced into various existing DNA molecules (or such as vectors) and cells known in the art. Furthermore, mutations can be introduced into the protein sequence of the present invention by chemical synthesis.

The present invention also relates to vectors containing the appropriate DNA sequence and appropriate promoters or control sequences mentioned above. These vectors can be used to transform appropriate host cells to enable them to express the protein.

Host cells can be prokaryotic cells, such as bacterial cells; or lower eukaryotic cells, such as yeast cells; or higher eukaryotic cells, such as mammalian cells. Representative examples include: bacterial cells of *Escherichia coli, Streptomyces, Salmonella typhimurium;* fungal cells such as yeast; insect cells of *Drosophila* S2 or Sf9; animal cells such as CHO, COS7, 293 cells, etc.

Transformation of host cells with recombinant DNA can be performed using conventional techniques well known to those skilled in the art. When the host is a prokaryote such as *E. coli,* competent cells capable of DNA uptake can be harvested after the exponential growth phase and treated with the CaCl₂ method, the procedures used are well known in the art. Another method is to use MgCl₂. If necessary, transformation can also be performed by electroporation. When the host is a eukaryote, DNA transfection methods such as calcium phosphate co-precipitation, conventional mechanical methods such as microinjection, electroporation, liposome packaging, etc. can be used.

The obtained transformants can be cultured by conventional methods to express the polypeptide encoded by the gene of the present invention. Depending on the host cell used, the culture medium can be selected from various conventional media. Cultivation is carried out under conditions suitable for host cell growth. When the host cells grow to an appropriate cell density, the selected promoter is induced by a suitable method (such as temperature shift or chemical induction), and the cells are cultured for an additional period.

The recombinant polypeptide in the above method can be expressed intracellularly, on the cell membrane, or secreted outside the cell. If desired, the recombinant protein can be separated and purified by various separation methods based on its physical, chemical, and other properties. These methods are well known to those skilled in the art. Examples of these methods include, but are not limited to: conventional renaturation treatment, treatment with protein precipitants (salting-out methods), centrifugation, osmotic disruption, sonication, ultracentrifugation, molecular sieve chromatography (gel filtration), adsorption chromatography, ion exchange chromatography, high performance liquid chromatography (HPLC) and other various liquid chromatography techniques and combinations of these methods.

The single domain antibodies or antibodies of the present invention can be used alone, or can be combined or conjugated with detectable labels (for diagnostic purposes), therapeutic agents, PK (protein kinase) modifying moieties, or any combination of the above.

Detectable labels for diagnostic purposes include, but are not limited to: fluorescent or luminescent labels, radioactive labels, MRI (Magnetic Resonance Imaging) or CT (Computed Tomography) contrast agents, or enzymes capable of producing a detectable product.

Therapeutic agents that can be combined or conjugated with the antibodies of the present invention include, but are not limited to: 1. radionuclides; 2. biotoxins; 3. cytokines such as IL-2, etc.; 4. gold nanoparticles/nanorods; 5. viral particles; 6. liposomes; 7. magnetic nanoparticles; 8. prodrug-activating enzymes (e.g., DT-diaphorase (DTD) or biphenyl hydrolase-like protein (BPHL)), etc.

### Anti-TfR1 Single Domain Antibody/Nanobody

In the present invention, the anti-TfR1 single domain antibody includes monomers, bivalent (bivalent antibody), tetravalent (tetravalent antibody), and/or multivalent (multivalent antibody) forms.

In a preferred embodiment of the present invention, the amino acid sequence of the VHH chain of the anti-TfR1 single domain antibody is selected from one or more of SEQ ID NOs: 1-44 and 210-215.

The anti-TfR1 single domain antibodies of the present invention can specifically bind to human and/or alpaca TfR1, but do not bind or weakly bind to murine TfR1; can specifically bind to monkey TfR1, or do not bind or weakly bind to monkey TfR1.

The anti-TfR1 single domain antibodies of the present invention can block the binding of TfR1 to transferrin, or do not block the binding of TfR1 to transferrin, but do not bind to TfR2, and do not block the binding of TfR1 to HFE protein (HFE protein interacts with transferrin receptor, affecting the interaction between transferrin and transferrin receptor, and regulating iron balance in the body).

### Labeled Antibodies

In a preferred embodiment of the present invention, the single domain antibody or antibody may carry a detectable label. More preferably, the label is selected from the group consisting of: an isotope, a colloidal gold label, a colored label, or a fluorescent label.

Colloidal gold labeling can be performed using methods known to those skilled in the art. In a preferred embodiment of the present invention, the anti-TfR1 single domain antibody or antibody is labeled with colloidal gold to obtain a colloidal gold-labeled antibody. The anti-TfR1 single domain antibodies or antibodies of the present invention can effectively bind to TfR1 protein.

### Detection Methods

The present invention also relates to a method for detecting TfR1 protein or a fragment thereof. The method generally comprises the following steps: obtaining a cell and/or tissue sample; dissolving the sample in a medium; detecting the level of TfR1 protein in the dissolved sample.

In the detection method of the present invention, the sample used is not particularly limited, and a representative example is a cell-containing sample in a cell preservation solution.

### Kits

The present invention also provides a kit containing the anti-TfR1 single domain antibody or antibody (or fragment thereof) or a test strip of the present invention. In a preferred embodiment of the present invention, the kit further comprises a container, instructions for use, buffers, etc.

The present invention also provides a detection kit for detecting TfR1 protein levels, which includes an antibody that recognizes TfR1 protein, a lysis medium for dissolving the sample, and general reagents and buffers required for detection, such as various buffers, detection labels, detection substrates, etc. The detection kit can be an *in vitro* diagnostic device.

### Pharmaceutical Compositions

The present invention also provides a pharmaceutical composition. It comprises, as an active ingredient, the anti-TfR1 single domain antibody of the first aspect of the present invention, the anti-TfR1 antibody of the second aspect of the present invention, the chimeric antigen receptor of the third aspect of the present invention, the fusion protein of the fourth aspect of the present invention, the recombinant protein of the fifth aspect of the present invention, the host cell of the eighth aspect of the present invention, the engineered immune cell of the ninth aspect of the present invention, the immunoconjugate of the eleventh aspect of the present invention, or combinations thereof, and a pharmaceutically acceptable carrier.

Typically, these substances can be formulated in a non-toxic, inert, and pharmaceutically acceptable aqueous carrier medium, with a pH usually of about 5-8, preferably about 6-8, although the pH may vary depending on the nature of the formulated substance and the condition to be treated. The formulated pharmaceutical composition can be administered by conventional routes, including (but not limited to): intraperitoneal, intravenous, or topical administration.

The pharmaceutical composition of the present invention contains a safe and effective amount (e.g., 0.001-99 wt%, preferably 0.01-90 wt%, more preferably 0.1-80 wt%) of the above-described antibody (or conjugate thereof) of the present invention and a pharmaceutically acceptable carrier or excipient. Such carriers include (but are not limited to): saline, buffer, glucose, water, glycerol, ethanol, and combinations thereof. The pharmaceutical formulation should match the mode of administration. The pharmaceutical composition of the present invention can be prepared in the form of an injection, for example, by conventional methods using physiological saline or an aqueous solution containing glucose and other adjuvants. The pharmaceutical composition, such as an injection or solution, should be manufactured under sterile conditions. The dosage of the active ingredient is a therapeutically effective amount, for example, about 10 µg/kg body weight to about 50 mg/kg body weight per day. In addition, the polypeptide of the present invention can also be used together with other therapeutic agents.

When using the pharmaceutical composition, a safe and effective amount of the immunoconjugate is administered to a mammal, wherein the safe and effective amount is usually at least about 10 µg/kg body weight, and in most cases does not exceed about 50 mg/kg body weight, preferably the dose is about 10 µg/kg body weight to about 10 mg/kg body weight. Of course, the specific dose should also consider factors such as the route of administration and the patient's health condition, which are within the skill of the skilled physician.

### Applications

As described above, the single domain antibodies and antibodies of the present invention have a wide range of biological and clinical application values, and their applications relate to multiple fields such as diagnosis and treatment of diseases, basic medical research, and biological research related to TfR1 protein. A preferred application is for clinical diagnosis, prevention, and treatment targeting TfR1 protein.

The present invention also provides a method for stimulating T cell-mediated immune responses targeting mammalian tumor cell populations or tissues, comprising the step of administering the CAR-T cells of the present invention to a mammal.

In one embodiment, the present invention includes a type of cell therapy, where patient autologous T cells (or allogeneic donors) are isolated, activated, and genetically modified to produce CAR-T cells, which are then infused back into the same patient. This approach minimizes the incidence of graft-versus-host disease, and antigens are recognized by T cells in an MHC-unrestricted manner. Furthermore, a single CAR-T can treat all cancers expressing that antigen. Unlike antibody therapy, CAR-T cells can replicate *in vivo,* resulting in long-term persistence that can lead to sustained tumor control.

In one embodiment, the CAR-T cells of the present invention can undergo stable *in vivo* expansion and can persist for months to years. Additionally, CAR-mediated immune responses can be part of an adoptive immunotherapy procedure, wherein CAR-T cells can induce specific immune responses against tumor cells highly expressing the antigen recognized by the CAR antigen-binding domain. For example, the CAR-T cells of the present invention elicit specific immune responses against tumor cells highly expressing TfR1.

Cancers that can be treated include tumors that are not vascularized or substantially not yet vascularized, as well as vascularized tumors. Types of cancer treated with the CAR of the present invention include, but are not limited to: breast cancer, gastric cancer, colorectal cancer, ovarian cancer, lung cancer, prostate cancer, liver cancer, kidney tumors, small intestine cancer, large intestine cancer, bile duct cancer, cervical cancer, lymphoma, esophageal cancer, etc.

Generally, cells activated and expanded as described herein can be used to treat and prevent diseases such as tumors. Accordingly, the present invention provides methods of treating cancer comprising administering to a subject in need thereof a therapeutically effective amount of the CAR-T cells of the present invention.

The CAR-T cells of the present invention can be administered alone or as a pharmaceutical composition in combination with diluents and/or with other components such as IL-2, IL-17, or other cytokines or cell populations. Briefly, the pharmaceutical composition of the present invention may comprise the target cell population as described herein, in combination with one or more pharmaceutically or physiologically acceptable carriers, diluents, or excipients.

The pharmaceutical composition of the present invention can be administered in a manner suitable for the disease to be treated (or prevented). The amount and frequency of administration will be determined by factors such as the patient's condition, the type and severity of the patient's disease, or can be determined by clinical trials.

When indicating an "immunologically effective amount", "anti-tumor effective amount", "tumor-inhibiting effective amount" or "therapeutic amount", the precise amount of the composition of the present invention to be administered can be determined by a physician, considering the patient's (subject's) age, weight, tumor size, extent of infection or metastasis, and individual differences in condition. The pharmaceutical composition comprising the T cells described herein can be administered at a dose of 10⁴ to 10⁹ cells/kg body weight, preferably 10⁵ to 10⁷ cells/kg body weight (including all integer values within the range). T cell compositions can also be administered multiple times at these doses. Cells can be administered using infusion techniques well known in immunotherapy (see, e.g., Rosenberg et al., New Eng. J. of Med. 319:1676, 1988). The optimal dosage and treatment regimen for a particular patient can be readily determined by a medical practitioner skilled in the art by monitoring the patient's signs of disease and adjusting treatment accordingly.

Administration of the subject compositions can be in any convenient manner, including by nebulization, injection, swallowing, infusion, implantation or transplantation. The compositions described herein can be administered to a patient subcutaneously, intradermally, intratumorally, intranodally, intraspinally, intramuscularly, by intravenous injection or intraperitoneally. In one embodiment, the T cell compositions of the present invention are administered to a patient by intradermal or subcutaneous injection. In another embodiment, the T cell compositions of the present invention are preferably administered by intravenous injection. T cell compositions can be injected directly into a tumor, lymph node, or site of infection.

In certain embodiments of the invention, cells activated and expanded using methods described herein or other methods known in the art to expand T cells to therapeutic levels are administered to a patient in combination (e.g., before, simultaneously, or after) with any number of relevant treatment modalities, including but not limited to treatment with agents such as antiviral agents, cidofovir and interleukin-2, cytarabine (also known as ARA-C) or natalizumab for MS patients or efalizumab for psoriasis patients or other treatments for PML patients. In further embodiments, the T cells of the present invention may be used in combination with: chemotherapy, radiation, immunosuppressive agents such as cyclosporine, azathioprine, methotrexate, mycophenolate mofetil, and FK506, antibodies or other immunotherapeutic agents. In further embodiments, the cell compositions of the present invention are administered to a patient in combination (e.g., before, simultaneously, or after) with bone marrow transplantation, chemotherapeutic agents such as fludarabine, external beam radiation therapy (XRT), cyclophosphamide. For example, in one embodiment, the subject may undergo standard treatment with high-dose chemotherapy followed by peripheral blood stem cell transplantation. In some embodiments, the subject receives an infusion of the expanded immune cells of the present invention after transplantation. In an additional embodiment, the expanded cells are administered before or after surgery.

The dosage of the above treatments administered to a patient will vary with the precise nature of the condition being treated and the recipient of the treatment. The dosage ratio for human administration can be implemented according to accepted practices in the art. Typically, per treatment or per course of treatment, 1×10⁵ to 1×10¹⁰ of the modified T cells of the present invention can be administered to the patient, for example, by intravenous infusion.

### Main Advantages of the Present Invention

1. The present invention provides single domain antibodies targeting TfR1, which can specifically target the surface of cells highly expressing TfR1, including tumor cells, muscle cells, and brain endothelial cells. Their binding epitopes can be different from transferrin, and they do not affect the function of TfR1 in transporting iron ions. They can serve as a drug delivery vehicle, precisely targeting tumor and muscle cells and helping to penetrate the blood-brain barrier. Therefore, these TfR1-targeting single domain antibodies have great potential for the development of anti-tumor drugs and drugs targeting muscle and brain diseases.
2. The binding epitopes of the anti-TfR1 single domain antibodies of the present invention can also be the same as that of transferrin, and can be used for the treatment and/or diagnosis of specific diseases such as iron deficiency anemia caused by elevated transferrin levels.
3. The anti-TfR1 single domain antibodies of the present invention are non-competitive with HFE protein. HFE protein does not affect the binding of the anti-TfR1 single domain antibody and its humanized VHH-Fc of the present invention to human TfR1. The anti-TfR1 single domain antibodies of the present invention also do not affect the interaction of HFE protein with transferrin receptors (including TfR1). Therefore, drugs or preparations based on the anti-TfR1 single domain antibodies of the present invention are less affected by the *in vivo* environment and have a lower possibility of off-target effects.

The present invention will be further illustrated below with reference to the specific examples. It should be understood that these examples are only to illustrate the present invention, not to limit the scope of the present invention. The conditions of the experimental methods not specifically indicated in the following examples are usually in accordance with conventional conditions as described in e.g., Sambrook et al., Molecular Cloning: A Laboratory Manual (New York: Cold Spring Harbor Laboratory Press, 1989), or according to the conditions recommended by the manufacturers. Percentages and parts are calculated by weight unless otherwise stated.

### Example 1: Immunization of Alpacas to Induce Production of Anti-TfR1 Single Domain Antibodies and Serum Titer Detection

For the primary immunization, 0.5 mg of human TfR1 antigen (SinoBiological, Cat#11020-H07H) was mixed with an equal volume of Complete Freund's Adjuvant (CFA) and injected subcutaneously into healthy alpacas to stimulate the alpaca's immune system to produce corresponding anti-TfR1 antibodies. For the second booster immunization, 0.25 mg of human TfR1 antigen was mixed with an equal volume of Incomplete Freund's Adjuvant (IFA) and injected subcutaneously into the alpacas 20 days after the first immunization. Subsequently, the third and fourth booster immunizations were performed at 20-day intervals, using the same antigen dosage and adjuvant as the second immunization. Blood was collected one week after the second, third, and fourth immunizations, respectively, to detect the target antibody titers in the serum.

Serum titer detection was performed using ELISA plates coated and immobilized with TfR1 antigen proteins from three species: human, rhesus macaque, and mouse. The titers of the corresponding antibodies against different species of antigens were determined using an HRP-conjugated goat anti-alpaca secondary antibody. The serum antibody titer detection results after the second, third, and fourth immunizations are presented in Tables 1 to 3, respectively.

**Table 1 - Serum titer detection after the second immunization**

| Dilution Factor | Pre-immunization | | | Second Immunization | | |
|---|---|---|---|---|---|---|
| | Human TfR1 | Rhesus TfR1 | Murine TfR1 | Human TfR1 | Rhesus TfR1 | Murine TfR1 |
| 1:2K | 0.0459 | 0.0685 | 0.0611 | 1.7451 | 0.1677 | 0.0904 |
| 1:4K | 0.0452 | 0.0691 | 0.0548 | 1.0754 | 0.1107 | 0.0813 |
| 1:8K | 0.0450 | 0.0547 | 0.0502 | 0.6068 | 0.0771 | 0.0749 |
| 1:16K | 0.0435 | 0.0518 | 0.0470 | 0.5190 | 0.0682 | 0.0527 |
| 1:32K | 0.0500 | 0.0397 | 0.0459 | 0.3939 | 0.0491 | 0.0643 |
| 1:64K | 0.0465 | 0.0449 | 0.0452 | 0.3271 | 0.0533 | 0.0533 |
| 1:128K | 0.0464 | 0.0444 | 0.0450 | 0.0864 | 0.0514 | 0.0514 |
| BLANK | 0.0451 | 0.0453 | 0.0435 | 0.0478 | 0.0456 | 0.0456 |

**Table 2 - Serum titer detection after the third immunization**

| Dilution Factor | Second Immunization | | | Third Immunization | | |
|---|---|---|---|---|---|---|
| | Human TfR1 | Rhesus TfR1 | Murine TfR1 | Human TfR1 | Rhesus TfR1 | Murine TfR1 |
| 1:2K | 2.4338 | 0.2439 | 0.0881 | 4.0366 | 0.7197 | 0.1125 |
| 1:4K | 1.4075 | 0.1693 | 0.0781 | 3.6977 | 0.4265 | 0.0914 |
| 1:8K | 0.8946 | 0.0527 | 0.0111 | 3.0080 | 0.2613 | 0.0812 |
| 1:16K | 0.4964 | 0.0515 | 0.0665 | 2.2899 | 0.1819 | 0.0665 |
| 1:32K | 0.3248 | 0.0581 | 0.0598 | 1.5559 | 0.1437 | 0.0512 |
| 1:64K | 0.2090 | 0.0582 | 0.0487 | 0.9100 | 0.1268 | 0.0506 |
| 1:128K | 0.1786 | 0.0543 | 0.0554 | 0.5913 | 0.1190 | 0.0511 |
| BLANK | 0.0427 | 0.0663 | 0.0514 | 0.0514 | 0.0696 | 0.0502 |

**Table 3 - Serum titer detection after the fourth immunization**

| Dilution Factor | Third Immunization | | | Fourth Immunization | | |
|---|---|---|---|---|---|---|
| | Human TfR1 | Rhesus TfR1 | Murine TfR1 | Human TfR1 | Rhesus TfR1 | Murine TfR1 |
| 1:2K | 3.9396 | 0.7887 | 0.3426 | 3.9131 | 1.5269 | 0.7186 |
| 1:4K | 3.7460 | 0.4131 | 0.1860 | 3.5404 | 0.8289 | 0.3977 |
| 1:8K | 3.1932 | 0.2717 | 0.1335 | 3.2672 | 0.5003 | 0.2324 |
| 1:16K | 2.4522 | 0.1720 | 0.1110 | 2.6540 | 0.2519 | 0.1667 |
| 1:32K | 1.6196 | 0.1166 | 0.0931 | 1.9623 | 0.1743 | 0.1144 |
| 1:64K | 0.9888 | 0.0912 | 0.0894 | 1.2410 | 0.1393 | 0.1062 |
| 1:128K | 0.5876 | 0.0728 | 0.0905 | 0.7671 | 0.1072 | 0.0822 |
| BLANK | 0.0972 | 0.0644 | 0.0860 | 0.0904 | 0.0747 | 0.0697 |

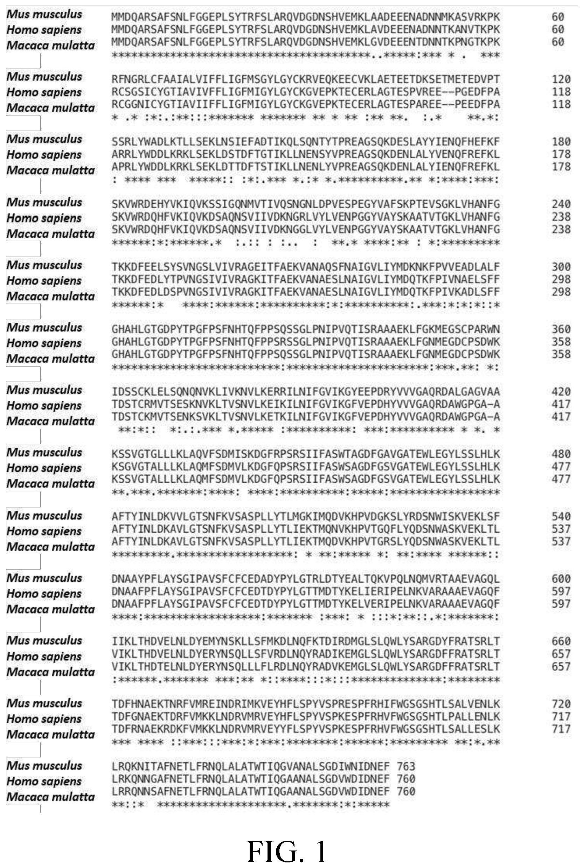

The serum titer detection results indicated that after immunization with human TfR1 antigen, the alpaca serum produced higher-titer anti-human TfR1 antibodies, moderate-titer anti-monkey TfR1 antibodies, and lower-titer anti-murine TfR1 antibodies. The protein sequence alignment results of TfR1 antigens from three species: human (*Homo sapiens*), rhesus monkey (*Macaca mulatta*)*,* and mouse (*Mus musculus*) showed that the human-monkey sequence similarity was 91%, and the human-murine sequence similarity was 76%. The sequence alignment results are shown in Figure 1. The alpaca serum immune antibody titers were as expected, and the peripheral blood after the fourth immunization could be used for antibody library construction.

### Example 2: Construction and Screening of Anti-TfR1 Single Domain Antibody Yeast Library

Peripheral blood mononuclear cells (PBMCs) were isolated from 50 mL of peripheral blood from alpacas after four immunizations, and total RNA was extracted using RNAiso Plus reagent. 5 µg of total RNA was reverse transcribed into cDNA using PrimeScript^{™} II 1st Strand cDNA Synthesis Kit (Takara, Cat#6210A), following the manufacturer's instructions. Using the cDNA as a template, 5 µl of 5-fold diluted cDNA was used for the first round of nested PCR. The PCR product fragment of about 750 bp was gel-purified and used as a template for the second round of nested PCR. After two rounds of nested PCR amplification, the single domain antibody fragments were obtained.

The PCR products were purified using the Cycle-Pure Kit and then co-transformed with linearized pYDC2 vector fragments into yeast competent cells by electroporation. The transformed cells were plated on 200 mm SD-CAA plates, with a total plating volume of 5 mL, and cultured at 30°C for 4 days.

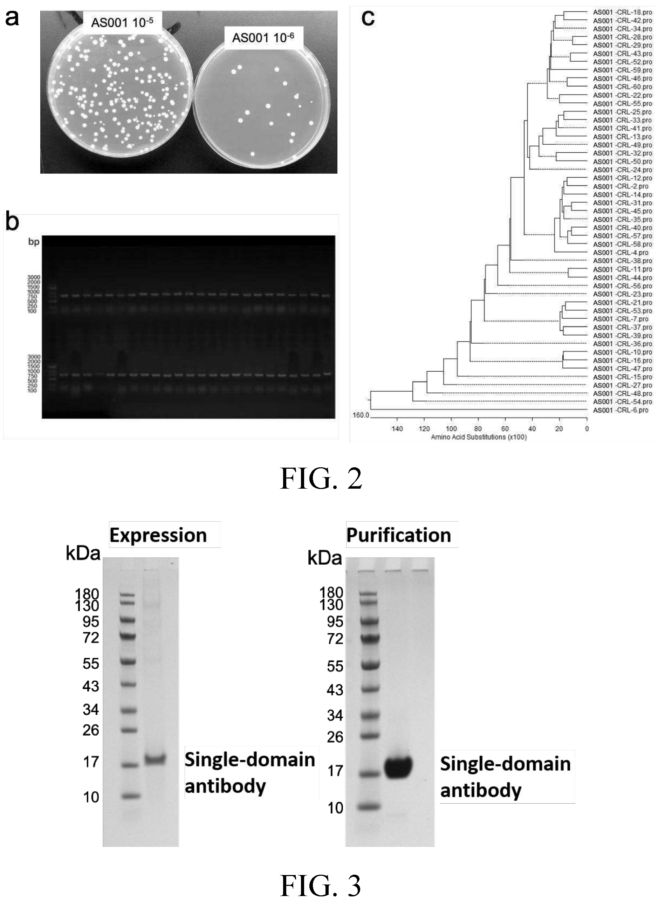

As shown in Figure 2 panel a, the library capacity was calculated by plating serial dilutions of 10 µL of the post-electroporation yeast cell suspension onto 90 mm plates. The total number of transformants was determined to be 1×10⁸ by the plate count method.

As shown in Figure 2 panel b, 48 single clones were randomly picked from the library plates for PCR identification, and the results showed a 100% insertion rate.

As shown in Figure 2 panel c, 60 randomly selected positive PCR products were sent for sequencing. The obtained sequences were translated into protein sequences, which were then subjected to amino acid sequence alignment and phylogenetic tree construction. Based on the number of transformants in the library, the library insertion rate, and the diversity analysis from sequencing, the capacity of the anti-TfR1 single domain antibody yeast library was determined to be 1×10⁸.

To obtain cross-reactive antibodies against both human and monkey species, the screening process used biotin-labeled rhesus monkey TfR1 antigen (SinoBiological, Cat#90253-C07H-B) for antibody screening and enrichment. The first round of enrichment was performed using 100 nM rhesus monkey TfR1 antigen by magnetic bead sorting, with a yeast cell input of 1.36×10¹⁰ cells. The second round of enrichment was performed on the magnetic bead-enriched library from the first round, using 100 nM rhesus monkey TfR1 antigen, to find the double-positive cell population by flow cytometry sorting, and to sort the double-positive single clones.

Subsequently, single clones were sequenced and subjected to sequence alignment analysis. Flow cytometry was used to further verify the binding activity of single clone cells corresponding to unique sequences to human, murine, and monkey TfR1 antigens. Based on sequence alignment and single clone flow cytometry binding activity verification analysis, a total of 44 sequences positive for human and monkey TfR1 antigen binding were obtained, and no sequences binding to murine TfR1 antigen were identified (as shown in Table 4). These sequences involved 23 types of CDR1, 25 types of CDR2, and 27 types of CDR3 sequences (as shown in Table 5).

**Table 4 - Human and Monkey TfR1 Antigen Binding-Positive Single Domain Antibody Sequences**

| Name | Amino Acid Sequence | SEQ ID NO: |
|---|---|---|
| TfR1-3 | QVQLVESGGGLVQPGGSLRLSCAASGFTFSTSTMTWVRQAPGK ELEWVSSIYKDGTLADYAGSVKGRFTISRDNAKNTLYLQMNSL KPEDTAVYYCAKGGPNYMNTNPKGQGTQVIVSS | 1 |
| TfR1-6 | QVQLVESGGGSVQTGGSLKLSCAASGFIFSSWSMSWVRQAPGK GLEWVSGFNNGDGSTRYADSVKGRFTISRDIAKNTMYLQMND LRSEDTAVYYCVRGEGLSWFDHWGQGTQVTVSS | 2 |
| TfR1-12 | EVQLVESGGGLVQAGGSLRLSCAASGSGFSVKTMAWYRRPPG KQHELLAVIGPGGSAKYVGSARGRFTVSRDNAKSTLYLQMTSL KPEDTAVYYCRESGTPNWGQGTQVTVSS | 3 |
| TfR1-13 | EVQLVESGGDLVQPGGSLRLSCAASGFTFSSYAMSWVRQAPG KGLEWVSGINSGGGSTSYADSVKGRFTISRDNAKNSVDLQMTS LKPEDTAIYYCAVDGSSWFDAVYWGQGTQVTVSS | 4 |
| TfR1-16 | QLQLVESGGGLVQPGGSLRLSCAASGFTFSSYTMSWLRQAPGK GLEWVSSINSGGGGTDYADSVKGRFTISRDNAKNTLYLQMNSL KPEDTAVYYCAMNGPNYYTESTRGQGTQVTVSS | 5 |
| TfR1-21 | EVQLVESGGGVVQTGGSLRLSCAASGFTFSKHVMSWLRQAPG KGLEWVSAINSDGSGTSYADSVKGRFTIARDNAKNTLYLQMN TLKPEDTAVYYCANRGPNVYSTTYYTKGQGTQVTVSS | 6 |
| TfR1-23 | EVQLVESGGGLVQPGGSLRLSCAASGFTFSKHVMSWLRQAPG KGLEWVSAINSDGSGTSYADSVKGRFTIARDNAKNTLYLQMN TLKPEDTAVYYCANRGPNVYSSTYYTKGQGTQVTVSS | 7 |
| TfR1-27 | QVQLVESGGGLVQAGGSLRLSCAASGRRFSSSDMGWYRQASG KEREFVANIKASGGSTNYADSVKDRFTISRDNRKNTVYLQMDS LTLEDTAVYYCRLRRRATSEAYWGQGTQVTVSS | 8 |
| TfR1-37 | QLQLVESGGGLVQSGGSLRLSCTVSGDTLAHYAIKWFRQAPGK EREGVSCISTREGNTYYSDSVKGRFTISRDNAKNTVYLQMNSL KPEDTAVYYCAADILCIVPDWDYWGQGTLVTVSS | 9 |
| TfR1-41 | EVQLVESGGGLVQPGGSLRLSCAASGFTFSSYAMSWVRQAPG KGLEWVSTINSGGGSTSYADSVKGRFTISRDNAKNTLYLQMNS LKPEDTAVYYCAADGSSWFDAVYWGQGTQVTVSS | 10 |
| TfR1-45 | EVQLVESGGGLVQAGGSLRLSCAASGFTSDYYAISWFRQAPGK EREGVCSISISDGSTVYADSVKGRFTISRDSAKNTVYLQMNSLK PEDTAVYYCAAVVLIKGSWIDLLLPSHMSDWGKGTQVTVSS | 11 |
| TfR1-72 | EAEAQLQVVESGGGLVQPGGSLSLSCAVSGFALDSYAIGWFRQ APGKQRELVASITGGDKTNYENSVKGRFTISRDSARNTAYLQM NDLKPEDTAIYYCGVRVSRRKRYDSWGQGTQVTVSS | 12 |
| TfR1-74 | EVQLVESGGGLVQSGGSLRLSCTVSGDTLAHYAIKWFRQAPGK EREGVSCISTREGNTYYSDSVKGRFTISRDNAKNTVYLQMNSL KPEDTAVYYCAADILCIVPDWDHWGQGTLVTVSS | 13 |
| TfR1-95 | QLQLVESGGGLVQPGGSLRLSCAASGFTFSSKTMSWLRQAPGK GLEWVSSINSGGSGTDYADSVKGRFTISRDNAKNTLYLQMNSL KPEDTAVYYCAINGPNYYRESTRGQGTQVTVSS | 14 |
| TfR1-105 | EVQVVESGGGLVHPGGSLRLSCAASGFTSDYYAISWFRQAPGK EREGVCSISISDGSTVYADSVKGRFTISRDSAKNTVYLQMNSLK PEDTAVYYCAAVVLIKGSWIDLLLPSHMSDWGKGTLVTVSS | 15 |
| TfR1-109 | EVQLVESGGGLVQPGGSLRLSCAASGFTFSSYTMSWLRQAPGK GLEWVSSINSGGGGTDYADSVKGRYTISRDNAKNTLYLQMSSL KPEDTAVYYCAMNGPNYYTESTRGQGTQVTVSS | 16 |
| TfR1-111 | QVQLVESGGGSVQPGGSLRLSCTASGFTFSSYTMSWLRQAPGK GLEWVSSINSSGGGTDYADSVKGRFTISRDNAKNTLYLQMNSL KPEDTAVYYCAMNGPNYYTESTRGQGTQVTVSS | 17 |
| TfR1-114 | QVQLVESGGGLVQPGGSLRLSCAASGFTFSSYTMSWVRQAPG KGLEWVSAINSGGGSTSYADSVKGRFTISRDNAKNTLYLQMNS LKPEDTAVYYCAMNGPNYYSESTRGQGTQVTVSS | 18 |
| TfR1-120 | EVQVVESGGGLVQPGGSLRLSCAASGFTFSSYTMSWLRQAPGK GLEWVSSINSGGGGTDYADSVKGRFTISRDNAKNTLYLQMNSL KPEDTAVYYCAMNGPNYYTESTRGQGTQVTVSS | 19 |
| TfR1-121 | EVQLVESGGGLVQPGGSLRLSCAASGFTFSKHVMSWLRQAPG KGLEWVSAINSDGSGTSYADSVKGRFTIARDNAKNTLYLQMN TLKPEDTAVYYCANRGPNVYSTTYYTKGQGTQVTVSG | 20 |
| TfR1-131 | EVQLVESGGGLVQSGGSLRLSCTVSGDTLAHYAIKWFRQAPGK EREGVSCISTREGNTYYSDSVKGRFTISRDNAKNTVYLQMNSL KPEDTAVYYCAADILCIVPDWDYWGQGTLVTVSS | 21 |
| TfR1-132 | QVQLVESGGGLVQAGGSLRLSCAASGFTFDDYALVWFRQAPG KEREGVSCISSRNGITYYADSVKGRFTISRDNAKNTVYLQMNSL KPEDTAVYYCAADPICLWTMTRGYEYDFWGQGTQVTVSS | 22 |
| TfR1-134 | EVQLVESGGGLVQPGGSLRLSCAASGFTFSGASMTWARQAPG KGLEWVSGIDRSGSSTQYADSVKGRFTISRDNAKNTLYLQMNS LKPEDTAVYYCARGMAWATRGQGTQVTVSS | 23 |
| TfR1-135 | EVQVVESGGGLVQAGDSLRLSCAVSGRRFSAYDMDWYRQAP GKEREFVARSRWRSGSKYTAHYADSVEGRFTISRSNGKNTVYL QMNSLKTEDTAIYYCNARDRGTVDSWGRGTQVTVSS | 24 |
| TfR1-401 | QVQLVESGGGLVQPGASLRLSCTASGLTFSDFTGHDMGWYRQ APGKKREFVAAISAKGDNTHYTDSVKGRFTIARDNAKSTVYLQ MLSLKPDDTAIYYCGVRVSRRKRYDSWGQGTQVTVSS | 25 |
| TfR1-415 | EVQLVESGGGTAQPGGSLRLSCAASGGSLERYDMGWYRQAPG KEREFVAGIRWSGGRKSYANSVKGRFTISRDNAKKTLYLQMNS LKPEDTAIYYCNAKTIRLNQKYVREYWGPGTQVTVSS | 26 |
| TfR1-429 | QVQLVESGGGLVQVGGSLRLSCVASGNFFILKTMGWYRQAPG KQRELVASITTKGTGGSANYADSVKGRFTISRDGTKNMVYLQ MNNLKPEDTAVYYCNARRKKDYWGQGTQVTVSS | 27 |
| TfR1-432 | QLQLVESGGGLVQPGGSLRLSCAASGFTFSSYTMSWLRQAPGK GLEWVSSINSGGTGTDAANSVKGRFTISRDNAKNTLYLQMNSL KPEDTAVYYCAMNGPNYYSESTRGQGTQVTVSS | 28 |
| TfR1-434 | EVQVVESGGGLVQPGGSLRLSCAASGFTFSSYTMSWVRQAPG KGLEWVSAINSGGGSTSYADSVKGRFTISRDNAKNTLYLQMNS LKPEDTAVYYCAMNGPNYYSESTRGQGTQVTVSS | 29 |
| TfR1-436 | QVQLVESGGGLVQPGGSLRLSCTASGRRFSSYDMGWYRQAPG KERELVAAITWTGKNTHYADSVKGRFTISRDTAKKTLYLQMNS LRSDDTAVYYCVRGWTYDIWGQGTQVTVSS | 30 |
| TfR1-446 | EVQVVESGGGLVQAGGSLRLSCTVSGDTLAHYAIKWFRQAPG KEREGVSCISTREGNTYYSDSVKGRFTISRDNAKNTVYLQMNS LKPEDTAVYYCAADILCIVPDWDYWGQGTLVTVSS | 31 |
| TfR1-449 | EVQLVESGGGLVHPGGSLRLSCAASGFTSDYYAISWFRQAPGK EREGVCSISISDGSTVYADSVKGRFTISRDSAKNTVYLQMNSLK PEDTAVYYCAAVVLIKGSWIDLLLPSHMSDWGKGTLVTVSS | 32 |
| TfR1-456 | QLQLVESGGGLVQAGGSLRLSCAVSGSRLSIKAMAWYRQAPG KQRELVAHIARGGGTTYTDSVKGRFTISRDSTKNTVVLQMNSL KPEDTAVYFCNAFPTSKHRSWGQGTQVTVSS | 33 |
| TfR1-528 | QVQLVESGGGLVQPGGSLRLSCAASGFTFSKHVMSWVRQAPG KALEWVSAINSDGSGTSYADSVKGRFTIARDNAKNTLYLQMNS LKPEDTAVYYCANRGPNVYSTTYYTKGQGTQVTVSS | 34 |
| TfR1-546 | QVQLVESGGGLVQAGGSLRLSCTASGSIWTINGMGWYRQAPD KQRELVASMASDGVTKYSDSVKGRFTISRDNAKNTVYLKMDS LKPEDTAIYYCHAEGTDYVGWNTHEYEYWGQGTQVTVSS | 35 |
| TfR1-547 | QLQLVESGGGLVQPGDSLRLSCTASGFTLDHYAIGWFRQAPGK EREGVSCISTREGNTYYSDSVKGRFTISRDNAKNTVYLQMNSL KPEDTAVYYCAADILCIVPDWDYWGQGTLVTVSS | 36 |
| TfR1-554 | EVQVVESGGGLVQPGGSLRLSCAASGFTFSKHVMSWLRQAPG KGLEWVSAINSDGSGTSYADSVKGRFTIARDNAEDTVYLQMN TLKPEDTAVYYCANRGPNVYSSTYYTKGQGTQVTVSS | 37 |
| TfR1-572 | QLQLVESGGGLVQAGGSLRLSCAASGSGFSVKTMAWYRQPPG KQRELLAVIGGGGSAKYVGSAKGRFTVSRDNARNTLYLQMNS LKPEDTAVYYCRESGSPYWGQGTQVTVSS | 38 |
| TfR1-584 | QVQLVESGGGLVQPGGSLRLSCAASGFTFSSYAMSWVRQAPG KGLEWVSSINKDDAVTAYSDSVKGRFTISRDAAKNTVYLQMN SLKPEDTAVYYCYAEEYYSADDYWGQGTQVTVSS | 39 |
| TfR1-589 | QLQLVESGGGLVQPGESLRLSCVVSAPTLNYYAISWFRQAPGK EREGVCSISISDGSTVYADSVKGRFTISRDSAKNTVYLQMNSLK PEDTAVYYCAAVVLIKGSWIDLLLPSHMSDWGKGTLVTVSS | 40 |
| TfR1-595 | QVQLVESGGGLVQPGGSLRLSCAASGFTFSSKTMSWLRQAPGK GLEWVSSINSGGGGTDYADSVKGRFTISRDNAKNTLYLQMNSL KPEDTAVYYCAINGPNYYSESTRGQGTQVTVSS | 41 |
| TfR1-597 | QVQLVESGGTLGQPGGSLRLSCAASGFAFSHFAMTWVRQAPG KGLEWVSTINYGGESTRYADSVKGRFTISRDNAKNTLYLQMN NLTPEDTAVYYCVRGEGLSWFDYWGQGTQVTVSS | 42 |
| TfR1-599 | QVQVVESGGGLVQPGGSLRLSCAASGFTFSGASMTWARQAPG KGLEWVSGIDRSGSSTQYADSVKGRFTISRDNAKNTLYLQMNS LKPEDTAVYYCARGMAWATRGQGTQVTVSS | 43 |
| TfR1-606 | EVQLVESGGGLVQAGGSLRLSCAASGFTFDDYALVWFRQAPG KEREGVSCISSRNGITYYADSVKGRFTISRDNAKNTVYLQMNSL KPEDTAVYYCAADPICLWTMTRGYEYDFWGQGTQVTVSS | 44 |

**Table 5 - Human and Monkey TfR1 Antigen Binding-Positive CDR Sequences**

| Antibody Name | CDR1 | SEQ ID NO: | Antibody Name | CDR2 | SEQ ID NO: | Antibody Name | CDR3 | SEQ ID NO: |
|---|---|---|---|---|---|---|---|---|
| TfR1-16, TfR1-109, TfR1-111, TfR1-114, TfR1-120, TfR1-432, TfR1-434 | GFTFSS YT | 45 | TfR1-16, TfR1-109, TfR1-120, TfR1-595 | INSG GGGT | 46 | TfR1-16, TfR1-109, TfR1-111, TfR1-120 | AMNGP NYYTES T | 47 |
| TfR1-13, TfR1-41, TfR1-584 | GFTFSS YA | 48 | TfR1-13, TfR1-41, TfR1-114, TfR1-434 | INSG GGST | 49 | TfR1-114, TfR1-432, TfR1-434 | AMNGP NYYSES T | 50 |
| TfR1-95 (AS001-N0 37-095-VH H1~ AS001-N0 37-095-VH H6), TfR1-595 | GFTFSS KT | 51 | TfR1-111 | INSS GGGT | 52 | TfR1-21, TfR1-121, TfR1-528 | ANRGPN VYSTTY YT | 53 |
| TfR1-21, TfRl-23, TfR1-121, TfR1-528, TfR1-554 | GFTFSK HV | 54 | TfR1-132, TfR1-606 | ISSRN GIT | 55 | TfR1-23, TfR1-554 | ANRGPN VYSSTY YT | 56 |
| TfRl-3 | GFTFSTS T | 57 | TfR1-37, TfR 1-74, TfR1-131, TfR1-446, TfR1-547 | ISTRE GNT | 58 | TfR1-429 | NARRK KDY | 59 |
| TfR1-134, TfR1-599 | GFTFSG AS | 60 | TfR1-21, TfRl-23, TfR1-121, TfR1-528, TfR1-554 | INSD GSGT | 61 | TfR1-72, TfR1-401 | GVRVSR RKRYDS | 62 |
| TfR1-132, TfR1-606 | GFTFDD YA | 63 | TfR 1-45, TfR1-105, TfR1-449, TfR1-589 | ISISD GST | 64 | TfR1-132, TfR1-606 | AADPIC LWTMT RGYEYD F | 65 |
| TfR1-597 | GFAFSH FA | 66 | TfR1-134, TfR1-599 | IDRS GSST | 67 | TfRl-37, TfR1-131, TfR1-446, TfR1-547 | AADILCI VPDWD Y | 68 |
| TfR 1-45, TfR1-105, TfR1-449 | GFTSDY YA | 69 | TfR1-12 | IGPG GSA | 70 | TfR1-134, TfR1-599 | ARGMA WAT | 71 |
| TfR1-589 | APTLNY YA | 72 | TfR1-95 (AS001-N0 37-095-VH H1~ AS001-N0 37-095-VH H6) | INSG GSGT | 73 | TfR 1-45, TfR1-105, TfR1-449, TfR1-589 | AAVVLI KGSWID LLLPSH MSD | 74 |
| TfR1-37, TfR1-74, TfR1-131, TfR1-446 | GDTLAH YA | 75 | TfRl-3 | IYKD GTLA | 76 | TfR1-3 | AKGGPN YMNTN P | 77 |
| TfR1-547 | GFTLDH YA | 78 | TfR1-6 | FNNG DGST | 79 | TfR1-6 | VRGEGL SWFDH | 80 |
| TfR1-6 | GFIFSS WS | 81 | TfR1-27 | IKAS GGST | 82 | TfR1-12 | RESGTP N | 83 |
| TfR1-436 | GRRFSS YD | 84 | TfR1-72 | ITGG DKT | 85 | TfR1-13 | AVDGSS WFDAV Y | 86 |
| TfR1-401 | GLTFSD FTGHD | 87 | TfR1-135 | SRWR SGSK YTA | 88 | TfR1-27 | RLRRRA TSEAY | 89 |
| TfR1-415 | GGSLER YD | 90 | TfR1-401 | ISAK GDNT | 91 | TfR1-41 | AADGSS WFDAV Y | 92 |
| TfR1-27 | GRRFSS SD | 93 | TfR1-415 | IRWS GGRK | 94 | TfR1-74 | AADILCI VPDWD H | 95 |
| TfR1-12, TfR1-572 | GSGFSV KT | 96 | TfR1-429 | ITTK GTGG SA | 97 | TfR1-95 (AS001-N0 37-095-VH H1~ AS001-N0 37-095-VH H6) | AINGPN YYREST | 98 |
| TfR1-456 | GSRLSIK A | 99 | TfR1-432 | INSG GTGT | 100 | TfR1-135 | NARDR GTVDS | 101 |
| TfR1-429 | GNFFIL KT | 102 | TfR1-436 | ITWT GKNT | 103 | TfR1-415 | NAKTIR LNQKY VREY | 104 |
| TfR1-546 | GSIWTI NG | 105 | TfR1-456 | IARG GGT | 106 | TfR1-436 | VRGWT YDI | 107 |
| TfR1-135 | GRRFSA YD | 108 | TfR1-546 | MAS DGVT | 109 | TfR1-456 | NAFPTS KHRS | 110 |
| TfR1-72 | GFALDS YA | 111 | TfR1-572 | IGGG GSA | 112 | TfR1-546 | HAEGTD YVGWN THEYEY | 113 |
| | | | TfR1-584 | INKD DAVT | 114 | TfR1-572 | RESGSP Y | 115 |
| | | | TfR1-597 | INYG GEST | 116 | TfR1-584 | YAEEYY SADDY | 117 |
| | | | | | | TfR1-595 | AINGPN YYSEST | 118 |
| | | | | | | TfR1-597 | VRGEGL SWFDY | 119 |

### Example 3: Construction of Yeast Expression Strain for Anti-TfR1 Single Domain Antibody and Protein Preparation

The anti-TfR1 single domain antibody gene sequence with a His tag added at the N-terminus was codon-optimized and constructed into the pPICZ alpha A plasmid. The plasmid was then linearized with PemI enzyme. 5 µL of the linearized plasmid was added to 100 µL of competent cells, and the plasmid carrying the target gene was transformed into X33 *Pichia pastoris* cells using an electroporator (Biorad, MicroPulser) with parameters set at 1.6 kV, 4 ms.

After electroporation, the *Pichia pastoris* cells were recovered in mixed medium (YPD : Sorbitol = 1:1). Then 50 µL of the *Pichia pastoris* cells were plated on YPD solid media containing 200, 400, 600, and 800 µg/mL zeocin (Invitrogen, Cat#R25001). High-copy strains of the target gene were selected by the gradient zeocin concentration. For single-clone screening, individual clones were cultured in GMGY medium (Sangon, Cat# B540130) at 30°C, 250 rpm. After obtaining sufficient cell biomass, GMMY medium (Sangon, Cat# B540131) was used to induce secretion and expression of the target single domain antibody at 20°C, 250 rpm. After 24 hours, 20 µL of supernatant was collected, and the expression levels of individual colonies were analyzed by gel electrophoresis. The strain with the highest expression level was selected for strain preservation and protein production.

Large-scale protein expression and purification were performed as follows: 400 µL of cell culture was inoculated into 200 mL of BMGY medium and cultured at 30°C, 250 rpm for 3 days for enrichment. The medium was then replaced with 80 mL of BMMY medium for induction, with 1% methanol supplemented every 24 hours. The target single domain antibody was induced for secretion at 20°C, 250 rpm for 3 days. After induction, the supernatant was collected by centrifugation at 12,000 rpm for 15 min in a refrigerated high-speed centrifuge, and the protein was purified using nickel ion affinity chromatography resin (Cytiva, Cat#17092108). Before use, the nickel ion affinity column was equilibrated with a binding buffer. The centrifuged supernatant containing the target protein was then loaded onto the column. The His-tagged protein was retained on the column by binding to nickel ions. Non-specifically bound impurities were subsequently washed away with a buffer containing 20 mM imidazole, and the target protein was finally eluted with an elution buffer containing 250 mM imidazole.

As shown in Figure 3, the SDS-PAGE gel indicated that the purity of the single domain antibody in the protein expression supernatant was greater than 95%, and after a single-step nickel ion affinity purification, the protein purity reached over 99%.

### Example 4: Binding Activity Analysis of Anti-TfR1 Single Domain Antibodies to Human and Monkey TfR1 Recombinant Proteins

Human TfR1-His (ACRO, Cat#CD1-H5243), monkey TfR1-His (ACRO, Cat#TFR-C524A), and murine TfR1-His (ACRO, Cat#TFR-M524B) antigen solutions were separately prepared in PBS at a final concentration of 1 µg/mL. Then, 100 µL of each solution was added to separate wells of a 96-well ELISA plate and coated overnight at 4°C. The plate was washed 3 times with PBST (PBS + 0.05% Tween 20), and 200 µL/well of blocking buffer (PBST + 3% BSA) was added and incubated at room temperature for 1 hour. After washing once with PBST, 100 µL/well of serially diluted single domain antibody solutions were added and incubated at room temperature for 1 hour. The plate was washed 3 times with PBST, and 100 µL/well of 1:5000 diluted horseradish peroxidase (HRP)-labeled rabbit anti-camelid VHH antibody solution (Genscript, Cat#A02016) was added and incubated at room temperature for 1 hour. After washing 3 times with PBST, the plate was blotted dry, and 100 µL/well of TMB chromogenic substrate solution (Beyotime, Cat#P0209) was added for color development at room temperature for 5 to 30 minutes, followed by the addition of 100 µL/well of stop solution (Beyotime, Cat#P0215). Absorbance at 450 nm of each well was measured using a microplate reader (Molecular Devices, SpectraMax i3x). The binding EC₅₀ values were fitted and calculated using a four-parameter sigmoidal curve equation in GraphPad Prism 9 software.

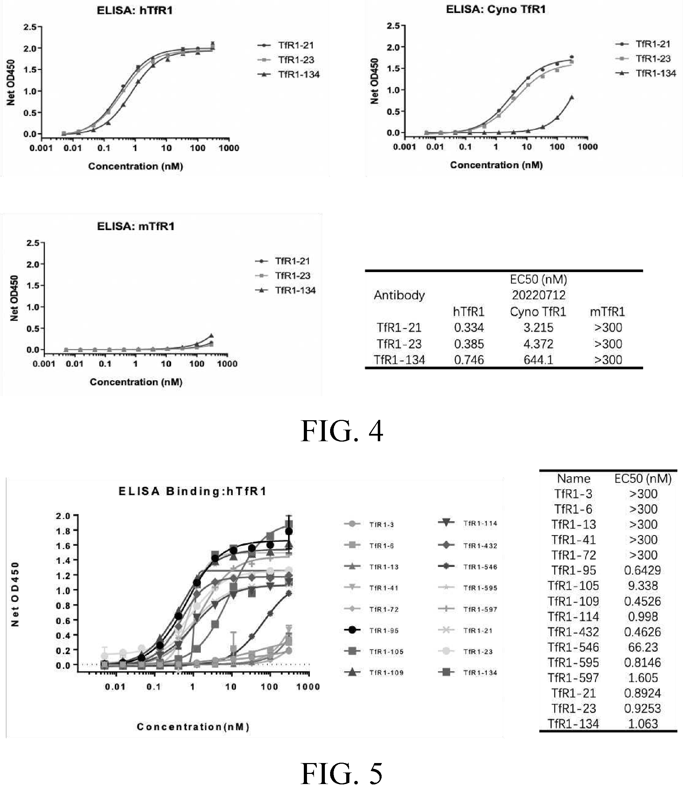

As shown in Figures 4 to 6, the experimental results indicated that most candidate antibodies exhibited cross-species binding activity to both human and monkey antigens, while none of the candidate antibodies showed binding activity to the murine antigen.

### Example 5: Holo-transferrin Does Not Affect the Binding Activity of Anti-TfR1 Single Domain Antibodies

Human TfR1-His (ACRO, Cat#CD1-H5243) antigen solution and human Holo-transferrin (Beyotime, Cat#ST1135) solution were separately prepared in PBS at final concentrations of 1 µg/mL and 100 µg/mL, respectively. Then, 100 µL of each solution was added to separate wells of a 96-well ELISA plate and coated overnight at 4°C. The plate was washed 3 times with PBST (PBS + 0.05% Tween 20), and 200 µL/well of blocking buffer (PBST + 3% BSA) was added and incubated at room temperature for 1 hour. After washing once with PBST, 100 µL/well of human TfR1-His solution at a concentration of 1 µg/mL was added to the wells coated with human Holo-transferrin and incubated at room temperature for 1 hour. After washing 3 times with PBST, 100 µL/well of serially diluted single domain antibody solutions were added to the wells coated with human TfR1-His and human Holo-transferrin, respectively, and incubated at room temperature for 1 hour. The plate was washed 3 times with PBST, and 100 µL/well of 1:5000 diluted horseradish peroxidase (HRP)-labeled rabbit anti-camelid VHH antibody solution (Genscript, Cat#A02016) was added and incubated at room temperature for 1 hour. After washing 3 times with PBST, the plate was blotted dry, and 100 µL/well of TMB chromogenic substrate solution (Beyotime, Cat#P0209) was added for color development at room temperature for 5 to 30 minutes, followed by the addition of 100 µL/well of stop solution (Beyotime, Cat#P0215). Absorbance at 450 nm of each well was measured using a microplate reader (Molecular Devices, SpectraMax i3x). The binding EC₅₀ values were fitted and calculated using a four-parameter sigmoidal curve equation in GraphPad Prism 9 software.

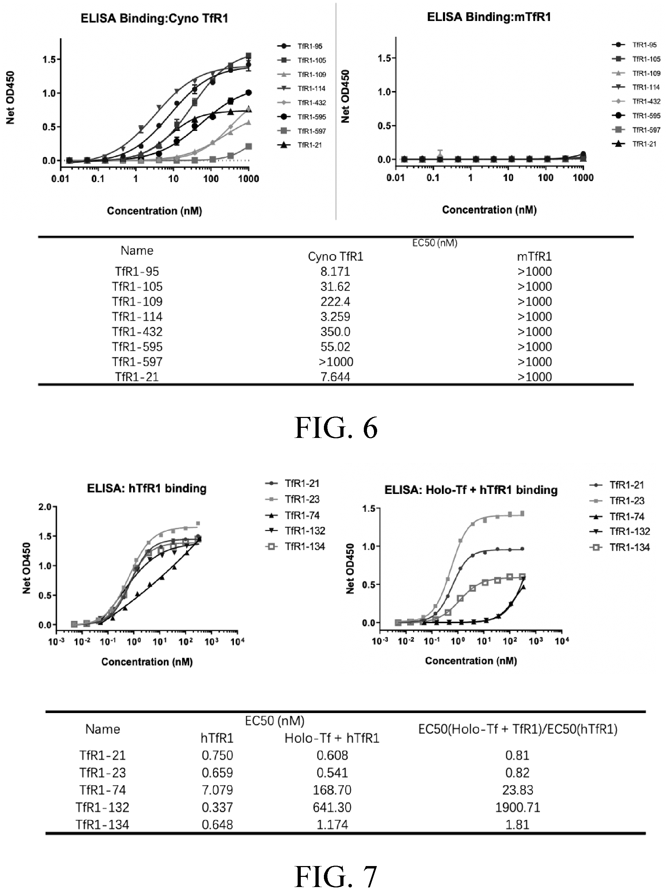

As shown in Figure 7, the experimental results indicated that candidate antibodies TfR1-21, TfR1-23, and TfR1-134 were non-competitive with Holo-transferrin and did not affect the iron-transporting function of TfR1; whereas candidate antibodies TfR1-74 and TfR1-132 were competitive with Holo-transferrin.

### Example 6: Binding Activity Analysis of Anti-TfR1 Single Domain Antibodies to Cells with High Human TfR Expression

The hTfR1 CHO-K1 cells and hTfR2 CHO-K1 cells were obtained by stably transfecting CHO-K1 cells with pIRES-Neo3 vectors expressing the human TfR1 gene (NM_003234.4) and human TfR2 gene (NM_003227.4), respectively. Cells in logarithmic growth phase were collected, washed with flow cytometry buffer (PBS + 2% FBS), and adjusted to a density of 1×10⁶ cells/mL. 180 µL/well of cell suspension was added to a 96-well M-bottom plate. Test sample stock solutions were diluted with flow cytometry buffer to prepare 10× serially diluted antibody solutions. 20 µL of the above solutions were added to the cell suspension in the 96-well plate, mixed, and incubated at 4°C for 30 minutes. After centrifugation at 4°C, 1000 rpm for 5 minutes, the supernatant was discarded, and the cells were washed twice with flow cytometry buffer. Then 200 µL/well of 1:1000 diluted iFluor647-labeled rabbit anti-camelid VHH antibody solution (Genscript, Cat#A02019) was added, mixed, and incubated at 4°C for 30 minutes. After centrifugation at 4°C, 1000 rpm for 5 minutes, the supernatant was discarded, and the cells were washed twice with flow cytometry buffer. Then the cells were resuspended in 200 µL/well of flow cytometry buffer. The mean fluorescence intensity of each sample was measured using a flow cytometer (BD, FACSCelesta). The binding EC₅₀ values were fitted and calculated using a four-parameter sigmoidal curve equation in GraphPad Prism 9 software.

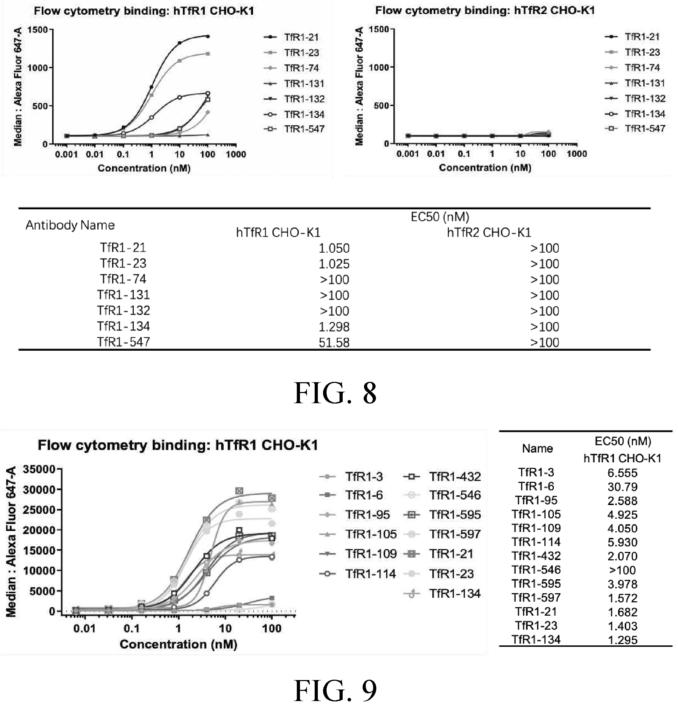

As shown in Figures 8 to 9, the experimental results indicated that most candidate antibodies can bind to cell-surface hTfR1, while none of the candidate antibodies bind to cell-surface hTfR2.

### Example 7: Humanization of Anti-TfR1 Single Domain Antibodies

The humanization of anti-TfR1 single domain antibodies was performed by aligning the parent sequence (AS001-N037-095-P, also referred to as TfR1-95, with the amino acid sequence set forth in SEQ ID NO: 14) against the human germline database. After defining the CDRs and framework regions of the parent antibody, sequences with varying degrees of humanization were designed based on the differential sites within the framework regions.

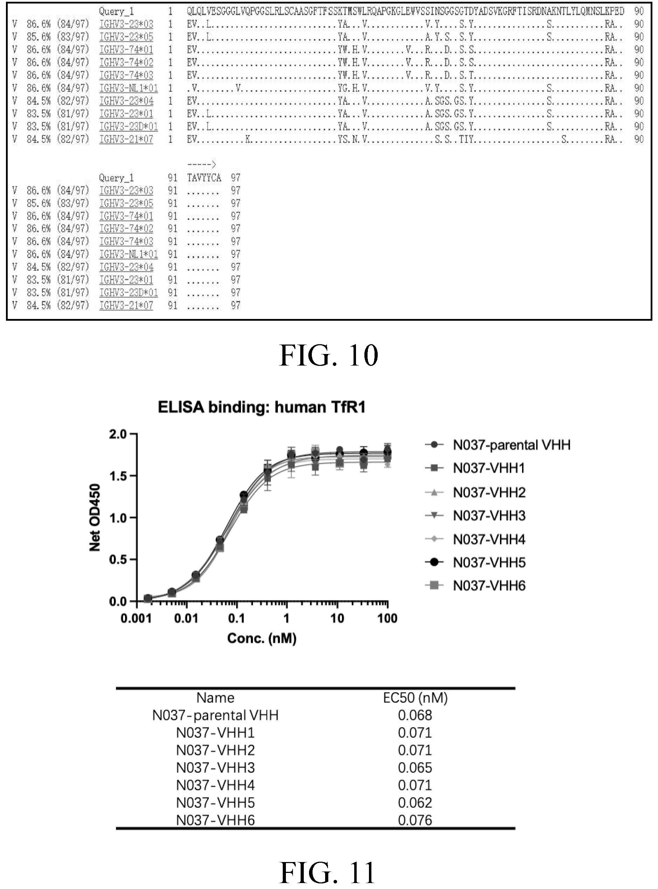

As shown in Figure 10, the sequence alignment results showed that the parental sequence had the highest homology with the human germline IGHV3-23 sequence, containing 8 camelid amino acid residues. Therefore, the IGHV3-23 with the highest homology was selected as the template for humanization design. Six sequences with more than 95% humanization degree (VHH1, VHH2, VHH3, VHH4, VHH5, VHH6) were designed by back-mutations, namely AS001-N037-095-VHH1 to AS001-N037-095-VHH6 in Table 6, whose amino acid sequences are set forth in SEQ ID NOs: 210-215, respectively.

**Table 6 - Summary of Humanized Sequences of Anti-TfR1 Single Domain Antibodies**

| Sample Name | Humanization Degree (%) | Sequence | SEQ ID NO: |
|---|---|---|---|
| AS001-N037-09 5-P (short for N037-parental VHH, P) | 93.3 | QLQLVESGGGLVQPGGSLRLSCAASGFTFSSKT MSWLRQAPGKGLEWVSSINSGGSGTDYADSV KGRFTISRDNAKNTLYLQMNSLKPEDTAVYYC AINGPNYYRESTRGQGTQVTVSS | 14 |
| AS001-N037-09 5-VHH1 (short for N037-VHH1, VHH1) | 95.0 | QLQLVESGGGLVQPGGSLRLSCAASGFTFSSKT MSWLRQAPGKGLEWVSSINSGGSGTDYADSV KGRFTISRDNAKNTLYLQMNSLKPEDTAVYYC AINGPNYYRESTWGQGTLVTVSS | 210 |
| AS001-N037-09 5-VHH2 (short for N037-VHH2, VHH2) | 95.8 | QLQLVESGGGLVQPGGSLRLSCAASGFTFSSKT MSWLRQAPGKGLEWVSSINSGGSGTDYADSV KGRFTISRDNAKNTLYLQMNSLRAEDTAVYYC AINGPNYYRESTWGQGTQVTVSS | 211 |
| AS001-N037-09 5-VHH3 (short for N037-VHH3, VHH3) | 95.8 | QLQLVESGGGLVQPGGSLRLSCAASGFTFSSKT MSWLRQAPGKGLEWVSSINSGGSGTDYADSV KGRFTISRDNAKNTLYLQMNSLRAEDTAVYYC AINGPNYYRESTRGQGTLVTVSS | 212 |
| AS001-N037-09 5-VHH4 (short for N037-VHH4, VHH4) | 96.6 | QLQLVESGGGLVQPGGSLRLSCAASGFTFSSKT MSWLRQAPGKGLEWVSSINSGGSGTDYADSV KGRFTISRDNAKNTLYLQMNSLRAEDTAVYYC AINGPNYYRESTWGQGTLVTVSS | 213 |
| AS001-N037-09 5-VHH5 (short for N037-VHH5, VHH5) | 97.5 | EVQLVESGGGLVQPGGSLRLSCAASGFTFSSKT MSWLRQAPGKGLEWVSSINSGGSGTDYADSV KGRFTISRDNAKNTLYLQMNSLRAEDTAVYYC AINGPNYYRESTRGQGTLVTVSS | 214 |
| AS001-N037-09 5-VHH6 (short for N037-VHH6, VHH6) | 98.3 | EVQLVESGGGLVQPGGSLRLSCAASGFTFSSKT MSWLRQAPGKGLEWVSSINSGGSGTDYADSV KGRFTISRDNAKNTLYLQMNSLRAEDTAVYYC AINGPNYYRESTWGQGTLVTVSS | 215 |

### Example 8: Expression and Purification of Humanized Anti-TfR1 VHH-Fc

The humanized VHH sequences were fused at the C-terminus to a human IgG1 Fc region. The fusion sequences were codon-optimized and constructed into the pcDNA3.4 vector. The fusion expression plasmid was transiently transfected into Expi CHO cells for 7 days of expression using ExpiCHO^{™} Expression Medium (Thermo Fisher, Cat# A2910001) and ExpiFectamine^{™} CHO Transfection Kit (Thermo Fisher, Cat# A29129). After expression, the cells were centrifuged, and the supernatant was collected, filtered through a 0.22 µm membrane, and purified using Protein A affinity resin (Cytiva, MabSelect SuRe^{™}) to obtain the humanized VHH-Fc proteins.

### Example 9: Stability Analysis of Humanized Anti-TfR1 VHH-Fc

The aggregation propensity of the humanized VHH-Fc was analyzed by HPLC-SEC. The chromatography column used in the experiments was XBridge BEH 200Å, SEC 3.5 µm, 7.8 × 300 mm (Waters, Cat# 186007640), with a flow rate of 0.8 mL/min. The detection wavelength was set at 280 nm.

The detection results are summarized in Table 7, which indicate that the monomer proportion of all humanized antibodies was greater than 98%, demonstrating that the samples were highly stable and exhibited a low tendency to form aggregates.

**Table 7 - Summary of HPLC-SEC Results for Humanized Anti-TfR1 VHH-Fc**

| Protein Name | Monomer Retention Time (min) | High Molecular Weight Aggregates (%) | Monomer (%) | Low Molecular Weight Species (%) |
|---|---|---|---|---|
| N037-parental VHH | 9.99 | / | 100.00 | / |
| N037-VHH1 | 10.09 | 0.70 | 99.30 | / |
| N037-VHH2 | 10.07 | 1.22 | 98.78 | / |
| N037-VHH3 | 10.02 | / | 100.00 | / |
| N037-VHH4 | 10.09 | 1.45 | 98.55 | / |
| N037-VHH5 | 9.97 | / | 100.00 | / |
| N037-VHH6 | 10.02 | / | 100.00 | / |

| | | | | |
|---|---|---|---|---|
| Note: "/" indicates none. | | | | |

Further thermal stability analysis of the humanized VHH-Fc was performed using Differential Scanning Fluorimetry (DSF). The instrument used in the experiments was an ABI7500 Fast Real-Time PCR system, and the experiment type was set to melt curve, in continuous mode, scanning over a temperature range of 25°C to 99°C. The first valley of the derivative of the melt curve was designated as the denaturation temperature Tm1 of the protein, and the second valley was designated as the denaturation temperature Tm2 of the protein. The detection results are shown in Table 8.

**Table 8 - Summary of DSF Results for Humanized Anti-TfR1 VHH-Fc**

| Protein Name | Tm1 (°C) | Tm2 (°C) |
|---|---|---|
| N037-parental VHH | 65.48 | / |
| N037-VHH1 | 59.97 | 66.51 |
| N037-VHH2 | 57.80 | 65.79 |
| N037-VHH3 | 65.48 | / |
| N037-VHH4 | 58.57 | 65.89 |
| N037-VHH5 | 66.77 | / |
| N037-VHH6 | 62.23 | 66.56 |

The results showed that VHH1, 2, 4, and 6 exhibited varying degrees of reduction in Tm1, indicating slightly lower stability than the parental molecule, while VHH3 had a Tm1 identical to the parental, and VHH5 showed a slightly higher Tm1 than the parental.

### Example 10: Binding Activity Analysis of Humanized VHH-Fc to Human and Monkey TfR1 Proteins

The binding activity of humanized VHH-Fc to human and monkey **TfR1** proteins was analyzed by Enzyme Linked Immunosorbent Assay (ELISA). Human TfR1-His (ACRO, Cat#CD1-H5243) and monkey TfR1-His (ACRO, Cat#TFR-C524a) antigen solutions were prepared in PBS at a final concentration of 1 µg/mL. Then, 25 µL/well of each solution was added to separate wells of a 384-well ELISA plate and incubated overnight at 4°C. The plate was washed 3 times with PBST (PBS + 0.05% Tween 20), and 50 µL/well of blocking buffer (PBST + 3% BSA) was added and incubated at room temperature for 1 hour. After washing once with PBST, 25 µL/well of serially diluted VHH-Fc solutions were added and incubated at room temperature for 1 hour. The plate was washed 3 times with PBST, and 25 µL/well of 1:5000 diluted horseradish peroxidase (HRP)-labeled goat anti-human IgG antibody solution (Yeasen, Cat#33501ES60) was added and incubated at room temperature for 1 hour. After washing 3 times with PBST, the plate was blotted dry, and 25 µL/well of TMB chromogenic substrate solution (Beyotime, Cat#P0209) was added for color development at room temperature for 5 to 30 minutes, followed by the addition of 25 µL/well of stop solution (Beyotime, Cat#P0215). Absorbance at 450 nm of each well was measured using a microplate reader (Molecular Devices, SpectraMax i3x). The binding EC₅₀ values were fitted and calculated using a four-parameter sigmoidal curve equation in GraphPad Prism 9 software.

As shown in Figures 11 to 12, the experimental results indicated that all humanized VHH-Fc proteins bound to human TfR1-His, with binding activity comparable to the parental, and VHH3 and VHH5 showed slightly stronger binding; VHH1, 2, 4, and 6 lost binding activity to monkey TfR1-His, while VHH3 and VHH5 retained monkey TfR1-His binding activity comparable to the parental.

Surface Plasmon Resonance (SPR) was used to analyze the binding activity of humanized VHH-Fc to human TfR1 protein (ACRO, Cat#CD1-H5243). The instrument used was a biacore T200 (Cytiva). The detection results are shown in Table 9.

**Table 9 - SPR Analysis Results of Anti-TfR1 Humanized VHH-Fc**

| Sample Name | Human TfR1 Protein | | | | |
|---|---|---|---|---|---|
| | K_{D} (M) | Kₐ (1/Ms) | K_{d} (l/s) | Rmax (nm) | Chi² |
| N037-parental VHH | 7.46E-10 | 4.06E+04 | 3.03E-05 | 1038 | 4.04 |
| N037-VHH1 | 6.13E-09 | 1.82E+04 | 1.12E-04 | 752.9 | 2.72 |
| N037-VHH2 | 5.96E-09 | 1.93E+04 | 1.15E-04 | 805.9 | 5.07 |
| N037-VHH3 | 6.23E-10 | 4.19E+04 | 2.61E-05 | 784.5 | 2.95 |
| N037-VHH4 | 5.94E-09 | 1.79E+04 | 1.06E-04 | 729.9 | 2.71 |
| N037-VHH5 | 7.16E-10 | 3.90E+04 | 2.79E-05 | 888.3 | 2.84 |
| N037-VHH6 | 5.50E-09 | 1.70E+04 | 9.35E-05 | 716.3 | 3.04 |

The affinity test results were consistent with those of the ELISA method: all humanized VHH-Fc bound to human TfR1-His with binding activity comparable to the parental, and VHH3 and VHH5 were slightly stronger than the parental.

### Example 11: Binding Activity Analysis of Humanized VHH-Fc to Cells with High Human TfR Expression

The hTfR1 CHO-K1 cells in logarithmic growth phase were collected, washed with flow cytometry buffer (PBS + 2% FBS), and adjusted to a density of 1×10⁶ cells/mL. 180 µL/well of cell suspension was added to a 96-well M-bottom plate. Test sample stock solutions were diluted with flow cytometry buffer to prepare 10× serially diluted antibody solutions. 20 µL of the above solutions were added to the cell suspension in the 96-well plate, mixed, and incubated at 4°C for 30 minutes. After centrifugation at 4°C, 1000 rpm for 5 minutes, the supernatant was discarded, and the cells were washed twice with flow cytometry buffer. Then 200 µL/well of 1:1000 diluted FITC-labeled mouse anti-human IgG antibody solution (Biolegend, Cat#410720) was added, mixed, and incubated at 4°C for 30 minutes. After centrifugation at 4°C, 1000 rpm for 5 minutes, the supernatant was discarded, and the cells were washed twice with flow cytometry buffer. Then the cells were resuspended in 200 µL/well of flow cytometry buffer. The mean fluorescence intensity of each sample was measured using a flow cytometer (BD, FACSCelesta). The binding EC₅₀ values were fitted and calculated using a four-parameter sigmoidal curve equation in GraphPad Prism 9 software.

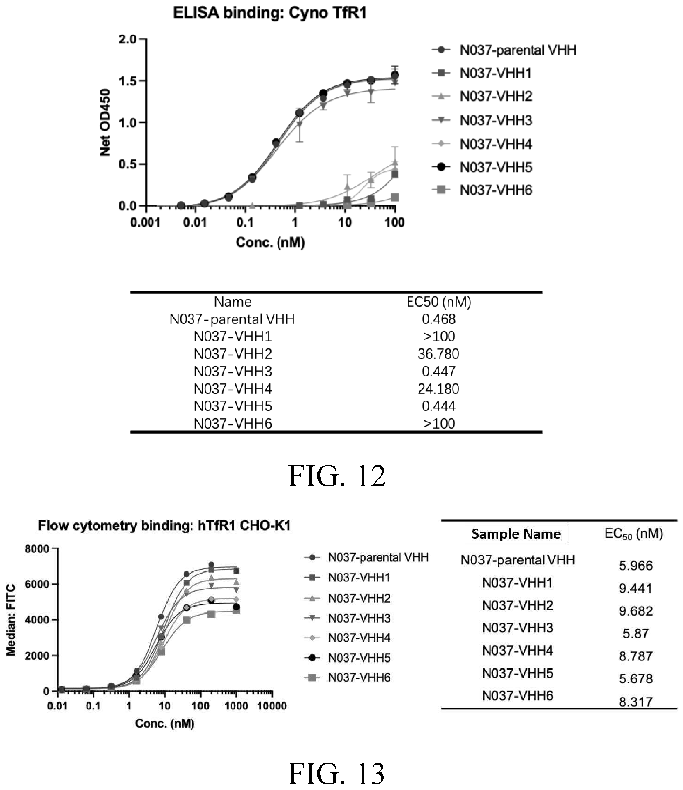

As shown in Figure 13, the experimental results indicated that all humanized VHH-Fc proteins bound to hTfR1 CHO-K1 cells. VHH3 and VHH5 showed binding activity comparable to the parental, while VHH1, 2, 4, and 6 showed slightly weaker binding activity than the parental.

### Example 12: Internalization Activity of Humanized VHH-Fc on SK-OV-3 Tumor Cells

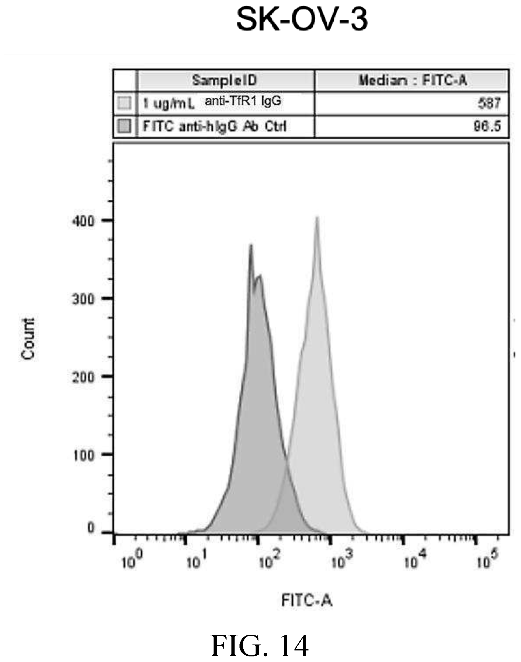

SK-OV-3 is a human ovarian cancer cell line. Flow cytometry analysis confirmed that TfR1 protein is highly expressed on its cell surface (see Figure 14 for the flow cytometry identification results). The internalization assay was performed using flow cytometry to evaluate the internalization activity of humanized VHH-Fc on SK-OV-3 tumor cells. The experimental procedure is as follows:
SK-OV-3 cells in logarithmic growth phase were collected, washed with flow cytometry buffer (PBS + 2% FBS), and adjusted to a density of 2×10⁶ cells/mL. For the blank and secondary antibody control groups, the cell density was adjusted to 5×10⁵ cells/mL. The cell suspensions were aliquoted into 1.5 mL centrifuge tubes at 1 mL/tube. After centrifugation at 4°C, 1000 rpm for 5 minutes, the supernatant was discarded, and the cells were resuspended in 1 mL of FITC-labeled mouse anti-human IgG antibody solution (Biolegend, Cat#410720) at a concentration of 15 µg/mL, and incubated at 4°C for 1 hour. After centrifugation at 4°C, 1000 rpm for 5 minutes, the supernatant was discarded, and the cells were washed twice with flow cytometry buffer. Then the cells were resuspended in 1 mL of complete medium (McCoy's 5A + 10% FBS) and aliquoted into Then the cells were resuspended in 1.5 mL centrifuge tubes at 250 µL/tube. The tubes were labeled as the no-wash group, no-internalization group, internalization-30 min group, and internalization-120 min group.

The no-wash and no-internalization groups were incubated on ice, while the internalization-30 min group and internalization-120 min group were incubated in a 37°C incubator for 30 min and 120 min, respectively. At the designated time points, the tubes were removed from the incubator and pre-chilled on ice for 5 min. All samples were centrifuged at 1000 rpm for 5 min at 4 °C, and the supernatant was discarded. The cells were washed once with flow cytometry buffer. Subsequently, the no-internalization, internalization-30 min, and internalization-120 min groups were centrifuged at 1000 rpm for 5 min at 4 °C, and the supernatant was discarded. The cells were resuspended in 250 µL/tube of elution buffer (0.05 M glycine, 0.1 M NaCl, pH adjusted to 2.5 with HCl) and incubated at room temperature for 8 min. Afterward, the cells were centrifuged at 1000 rpm for 5 min at 4 °C, and the supernatant was discarded, followed by two washes with flow cytometry buffer. All samples were centrifuged at 1000 rpm for 5 min at 4 °C, and the supernatant was discarded. The cells were resuspended in 200 µL/tube of fixation buffer (4% paraformaldehyde, pH 7.4) and incubated at 4 °C for 30 min.

The mean fluorescence intensity (MFI) of each sample was measured using a flow cytometer (BD, FACSCelesta) to calculate the percentage of antibody internalization. The detection window was calculated as: (MFI of the no-wash group - MFI of the blank control group) / (MFI of the no-internalization group - MFI of the blank cells). The percentage of antibody internalization was calculated as: (MFI of the internalization group - MFI of the no-internalization group) / (MFI of the no-wash group - MFI of the no-internalization group) × 100%. The results are shown in Tables 10 and 11.

**Table 10 - Summary of Internalization Results for Anti-TfR1 Humanized VHH-Fc**

| Sample Name | P-Fc | VHH1-Fc | VHH2-Fc | VHH3-Fc | VHH4-Fc | VHH5-Fc | VHH6-Fc |
|---|---|---|---|---|---|---|---|
| Secondary Ab Control MFI | 93.8 | 93.8 | 93.8 | 93.8 | 93.8 | 93.8 | 93.8 |
| No-Wash Group MFI | 657 | 622 | 647 | 671 | 584 | 703 | 561 |
| 0 h Internalization MFI | 191 | 148 | 153 | 181 | 146 | 198 | 152 |
| 2 h Internalization MFI | 462 | 425 | 457 | 451 | 407 | 491 | 395 |
| Detection Window | 5.79 | 9.75 | 9.34 | 6.62 | 9.39 | 5.85 | 8.03 |
| Elution Efficiency | 82.74% | 89.74% | 89.30% | 84.89% | 89.35% | 82.90% | 87.54% |
| 2 h Internalization Ratio | 58.15% | 58.44% | 61.54% | 55.10% | 59.59% | 58.02% | 59.41% |

**Table 11 - Summary of Internalization Results for Control IgG Antibody**

| Antibody Name | Median: FITC-A | | | | Internalization Percentage | | Detection Window |
|---|---|---|---|---|---|---|---|
| | No-Wash Group | 0 min Internalization | 30 min Internalization | 120 min Internalization | 30 min | 120 min | |
| 13E4_variant 2-ii | 731 | 206 | 292 | 300 | 16.4% | 17.9% | 6.41 |

The 2-hour internalization efficiency of the control hIgG1 antibody, 13E4_variant 2-ii (Avidity Bioscience, MS11028179), on SK-OV-3 cells was 17.9%, with a detection window of >6.41. In contrast, the 2-hour internalization efficiencies of the seven VHH-Fc antibodies on SK-OV-3 cells were all >55%, and their detection windows were all >5.7, indicating significantly higher internalization activity compared to the control antibody.

### Example 13: HFE Does Not Affect the Binding Activity of Humanized VHH-Fc

Human TfR1-His (ACRO, Cat#CD1-H5243) antigen solution and recombinant human HFE (Abcam, Cat#ab139241) solution were prepared in PBS at final concentrations of 1 µg/mL and 2 µg/mL, respectively. Then, 100 µL/well of each solution was added to separate wells of a 96-well ELISA plate and coated overnight at 4°C. The plate was washed 3 times with PBST (PBS + 0.05% Tween 20), and 200 µL/well of blocking buffer (PBST + 3% BSA) was added and incubated at room temperature for 1 hour. After washing once with PBST, 100 µL/well of human TfR1-His solution at a concentration of 1 µg/mL was added to the wells coated with human HFE and incubated at room temperature for 1 hour. After washing 3 times with PBST, 100 µL/well of serially diluted VHH-Fc solutions were added to the wells coated with human TfR1-His and human HFE, respectively, and incubated at room temperature for 1 hour. The plate was washed 3 times with PBST, and 100 µL/well of 1:5000 diluted horseradish peroxidase (HRP)-labeled goat anti-human IgG antibody solution (Yeasen, Cat#33501ES60) was added and incubated at room temperature for 1 hour. After washing 3 times with PBST, the plate was blotted dry, and 100 µL/well of TMB chromogenic substrate solution (Beyotime, Cat#P0209) was added for color development at room temperature for 5 to 30 minutes, followed by the addition of 100 µL/well of stop solution (Beyotime, Cat#P0215). Absorbance at 450 nm of each well was measured using a microplate reader (Molecular Devices, SpectraMax i3x). The binding EC₅₀ values were fitted and calculated using a four-parameter sigmoidal curve equation in GraphPad Prism 9 software.

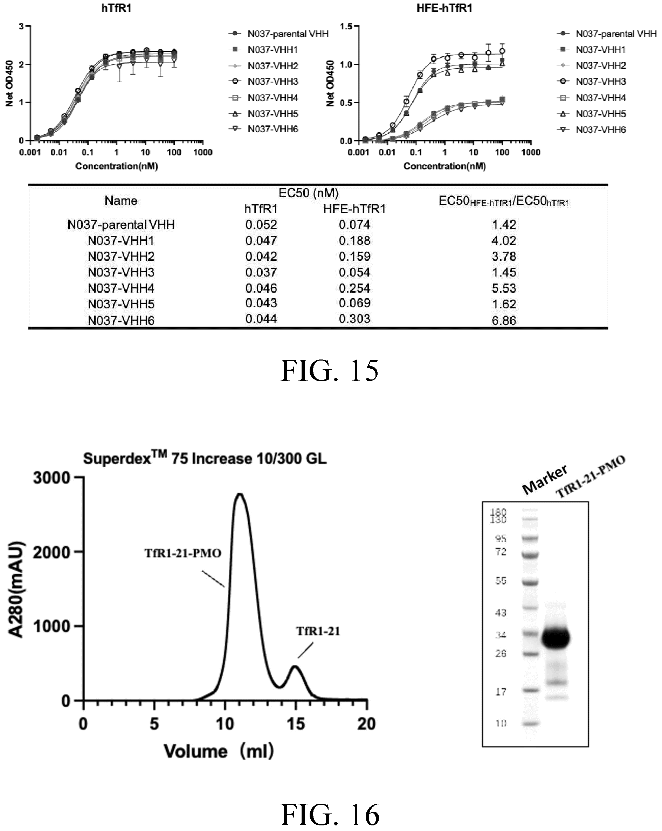

As shown in Figure 15, the experimental results indicated that HFE did not compete with humanized VHH-Fc, and had no effect on the binding of humanized VHH-Fc to human TfR1-His.

### Example 14: Preparation of Anti-TfR1 Single Domain Antibody-PMO Conjugates

Duchenne Muscular Dystrophy (DMD) is a genetic muscle wasting disease caused by mutations in the *Dystrophin* gene leading to the loss of Dystrophin protein. It manifests as progressive muscle weakness and atrophy. Patients typically begin to show symptoms around 2 to 5 years of age, lose ambulation by 9 to 12 years, and eventually the disease affects all smooth muscles, as well as cardiac and respiratory muscles, leading to respiratory failure or cardiac dysfunction and death. The average life expectancy for DMD patients is approximately 30 years.

PMO (Phosphorodiamidate morpholino oligomer) is a nucleic acid analog that can bind to pre-mRNA by complementary base pairing, causing the skipping of erroneous exons during RNA splicing, thereby producing a shortened but functional Dystrophin protein, thereby treating the disease.

In the present invention, a PMO sequence capable of inducing skipping of murine DMD exon 23 was selected, with the sequence set forth in SEQ ID NO: 218.

Exon 23 (SEQ ID NO: 218):
5'-GGCCAAACCTCGGCTTACCTGAAAT-3'

The 3' end of the PMO was modified with an NH₂ group for conjugation to the single domain antibody.

The PMO single strand with a 3'-terminal NH₂ modification was dissolved in phosphate buffer (50 mM NaH₂PO₄, 150 mM NaCl, pH 7.4) to prepare a stock solution at a final concentration of 1 mM. SM(PEG)₂ (linker molecule) powder was dissolved in dimethyl sulfoxide (DMSO) to freshly prepare a 250 mM SM(PEG)₂ stock solution. A 10- to 50-fold molar excess of the SM(PEG)₂ stock solution was added to the PMO single-strand stock solution. After rapid mixing, the reaction was carried out at room temperature for 30 min to 2 h. Upon completion of the reaction, 10% (v/v) of 1 M Tris-HCl (pH 7.0) was added to the reaction mixture. After thorough mixing, the mixture was incubated at room temperature for 20 min to quench further reaction of the excess SM(PEG)₂. Following incubation, unreacted SM(PEG)₂ linkers were removed by acetone precipitation to purify and obtain the SM(PEG)₂-PMO conjugate for subsequent use.

A cysteine mutation was introduced at the C-terminus of the anti-TfR1 single domain antibody TfR1-21 (SEQ ID NO: 6) for nucleic acid conjugation. The expression and purification methods for the single domain antibody were the same as those described in Example 3.

The single domain antibody was dialyzed against a dialysis buffer containing a reducing agent (20 mM Tris, 15 mM NaCl, pH 7.4). During dialysis, the C-terminal thiol groups were reduced, and small-molecule impurities such as free -SH groups were removed. The reduced single domain antibody and the SM(PEG)₂-PMO single strand were mixed at a molar ratio of 1:1 to 1:2. After thorough mixing, the reaction was carried out at room temperature for 2 h. A His-tag affinity column was used to remove unreacted SM(PEG)₂-PMO single strands, and the mixture containing the single domain antibody and the single domain antibody-PMO conjugate was collected. The single domain antibody and the single domain antibody-PMO conjugate were separated using a Superdex^{™} 75 Increase 10/300 GL column. The purity of the final product was verified by SDS-PAGE.

As shown in Figure 16, the experimental results demonstrated that after conjugation and two-step purification, a highly pure anti-TfR1 single domain antibody-PMO conjugate was obtained.

### Example 15: Analysis of DMD Exon-Skipping Efficacy of the Anti-TfR1 Single Domain Antibody-PMO Conjugate in hTfR1 Humanized Mice

The hTfR1 humanized C57BL/6 mice aged 6-8 weeks and weighing 16-24 g were selected. Unmodified PMO and the TfR1-21-PMO prepared in Example 14 were administered as a single intramuscular injection at a dose of 15 mg/kg PMO, with the PBS group serving as the control. Gastrocnemius muscle tissue samples were collected at 4, 7, and 14 days post-administration to analyze the percentage of exon skipping induced by the drug in the mouse gastrocnemius muscle tissue. A total of 27 mice were grouped by body weight and randomly divided into 3 groups, with 9 mice per group.

Detailed dosing information is provided in Table 12:

**Table 12 - Grouping and Dosing**

| Group | Test Article | Number of Animals (n) | Dose (mg/kg PMO) | Route of Administration | Dosing Frequency ^{a} |
|---|---|---|---|---|---|
| G1 | PBS | 9 | - | Intramuscular (i.m.) | Single dose |
| G2 | PMO | 9 | 15 | Intramuscular (i.m.) | Single dose |
| G3 | TfR1-21-PMO | 9 | 15 | Intramuscular (i.m.) | Single dose |

| | | | | | |
|---|---|---|---|---|---|
| Note: a: Single dose indicates a single administration on the day of grouping. | | | | | |

All mice were subjected to daily clinical observations, which included but were not limited to their general condition and food consumption. Body weight was measured twice a week. The monitoring data showed that the body weights of all mice fluctuated within the normal range during the experimental period, and no adverse reactions were observed. At the end of the study or upon reaching the humane endpoint, the animals were euthanized using an overdose of CO₂.

On days 4, 7, and 14 post-administration, bilateral gastrocnemius muscle tissue samples were collected from 3 mice in each group for exon-skipping analysis.

The collected tissues were homogenized for total RNA extraction and exon-skipping analysis. The homogenized tissue samples were lysed using TRIzol reagent (Thermo, Cat# 15596018), and total RNA was isolated using the phenol/chloroform method. Approximately 1 µg of each of the 72 RNA samples was subjected to reverse transcription (+gDNA wiper). The PCR program was run as follows: 37 °C for 45 min, followed by 85 °C for 5 sec. After reverse transcription, 2 µL of the cDNA product was used for the first-round PCR amplification with Ex20F Primer/Ex26R Primer (the reaction system and program are detailed in Table 13). Subsequently, 1 µL of the first-round PCR product was used for the second-round nested PCR with Ex20F2 Primer/Ex25R Primer (the reaction system and program are detailed in Table 14). Following the nested PCR, 10 µL of each sample was loaded onto a 3% TAE agarose gel for electrophoretic analysis. The expected size of the wild-type DMD product is 788 bp, and the expected size of the Exon 23-skipped DMD product is 575 bp.

The sequences of the primers used are as follows:
Ex20F: 5'-CAGAATTCTGCCAATTGCTGAG-3' (SEQ ID NO: 219)
Ex26R: 5'-TTCTTCAGCTTGTGTCATCC-3' (SEQ ID NO: 220)
Ex20F2: 5'-ACCCAGTCTACCACCCTATC-3' (SEQ ID NO: 221)
Ex25R: 5'-CTCTTTATCTTCTGCCCACCTT-3' (SEQ ID NO: 222)

**Table 13 - Primary PCR Protocol**

| | | | |
|---|---|---|---|
| Hot Start | 95°C | 2 min | 1 Cycle |
| Denaturation | 95°C | 0.5 min | 10 Cycles |
| Primer Annealing | 50°C | 0.5 min | |
| Primer Extension | 72°C | 1 min | |
| Final Extension | 72°C | 5 min | 1 Cycle |
| Hold | 4°C | - | - |

**Table 14 - Nested PCR Protocol**

| | | | |
|---|---|---|---|
| Hot Start | 95°C | 2 min | 1 Cycle |
| Denaturation | 95°C | 0.5 min | 35 Cycles |
| Primer Annealing | 50°C | 0.5 min | |
| Primer Extension | 72°C | 1 min | |
| Final Extension | 72°C | 5 min | 1 Cycle |
| Hold | 4°C | - | - |

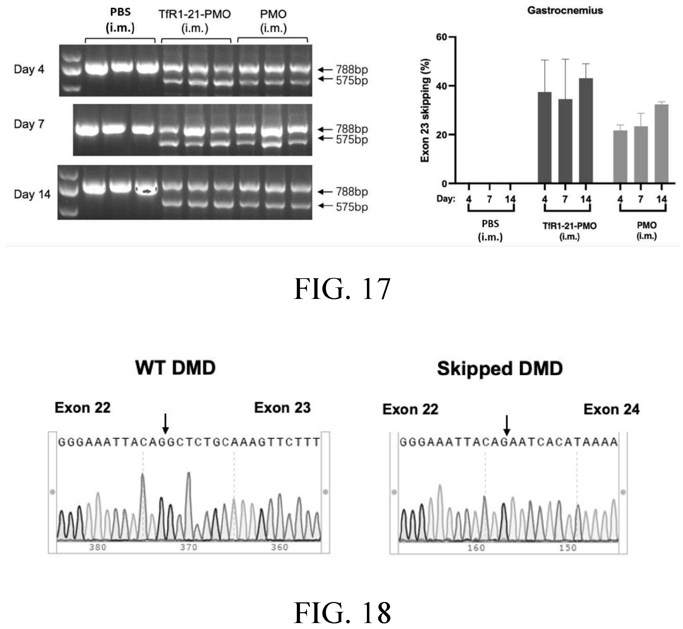

The agarose gel electrophoresis results were analyzed and quantified using Image Lab software, and bar charts were generated using GraphPad Prism 9 software. As shown in Figure 17, the experimental results demonstrated that the anti-TfR1 single domain antibody-PMO conjugate can effectively induce DMD exon 23 skipping in mouse gastrocnemius muscle tissue, and its exon-skipping efficacy is significantly superior to that of unmodified PMO.

The exon-skipping bands were excised from the gel for DNA sequencing to verify the success of exon skipping. As shown in Figure 18, the experimental results confirmed that DMD exon 23 skipping occurred correctly.

All literatures mentioned in the present invention are incorporated herein by reference, as though each one is individually incorporated by reference. **In** addition, it should be understood that, after reading the above teachings of the present invention, those skilled in the art can make various changes or modifications to the present invention, these equivalents also fall within the scope as defined in the appended claims of the present application.

## Claims

1. An anti-TfR1 single domain antibody, having one or more complementarity determining regions (CDRs) selected from the group consisting of:
(1) CDR1 as set forth in SEQ ID NO: 51, CDR2 as set forth in SEQ ID NO: 73, and CDR3 as set forth in SEQ ID NO: 98;
(2) CDR1 as set forth in SEQ ID NO: 54, CDR2 as set forth in SEQ ID NO: 61, and CDR3 as set forth in SEQ ID NO: 53;
(3) CDR1 as set forth in SEQ ID NO: 54, CDR2 as set forth in SEQ ID NO: 61, and CDR3 as set forth in SEQ ID NO: 56;
(4) CDR1 as set forth in SEQ ID NO: 60, CDR2 as set forth in SEQ ID NO: 67, and CDR3 as set forth in SEQ ID NO: 71;
(5) CDR1 as set forth in SEQ ID NO: 69, CDR2 as set forth in SEQ ID NO: 64, and CDR3 as set forth in SEQ ID NO: 74;
(6) CDR1 as set forth in SEQ ID NO: 45, CDR2 as set forth in SEQ ID NO: 49, and CDR3 as set forth in SEQ ID NO: 50;
(7) CDR1 as set forth in SEQ ID NO: 66, CDR2 as set forth in SEQ ID NO: 116, and CDR3 as set forth in SEQ ID NO: 119;
(8) CDR1 as set forth in SEQ ID NO: 45, CDR2 as set forth in SEQ ID NO: 100, and CDR3 as set forth in SEQ ID NO: 50;
(9) CDR1 as set forth in SEQ ID NO: 51, CDR2 as set forth in SEQ ID NO: 46, and CDR3 as set forth in SEQ ID NO: 118;
(10) CDR1 as set forth in SEQ ID NO: 45, CDR2 as set forth in SEQ ID NO: 46, and CDR3 as set forth in SEQ ID NO: 47;
(11) CDR1 as set forth in SEQ ID NO: 57, CDR2 as set forth in SEQ ID NO: 76, and CDR3 as set forth in SEQ ID NO: 77;
(12) CDR1 as set forth in SEQ ID NO: 81, CDR2 as set forth in SEQ ID NO: 79, and CDR3 as set forth in SEQ ID NO: 80;
(13) CDR1 as set forth in SEQ ID NO: 96, CDR2 as set forth in SEQ ID NO: 70, and CDR3 as set forth in SEQ ID NO: 83;
(14) CDR1 as set forth in SEQ ID NO: 48, CDR2 as set forth in SEQ ID NO: 49, and CDR3 as set forth in SEQ ID NO: 86;
(15) CDR1 as set forth in SEQ ID NO: 93, CDR2 as set forth in SEQ ID NO: 82, and CDR3 as set forth in SEQ ID NO: 89;
(16) CDR1 as set forth in SEQ ID NO: 75, CDR2 as set forth in SEQ ID NO: 58, and CDR3 as set forth in SEQ ID NO: 68;
(17) CDR1 as set forth in SEQ ID NO: 48, CDR2 as set forth in SEQ ID NO: 49, and CDR3 as set forth in SEQ ID NO: 92;
(18) CDR1 as set forth in SEQ ID NO: 111, CDR2 as set forth in SEQ ID NO: 85, and CDR3 as set forth in SEQ ID NO: 62;
(19) CDR1 as set forth in SEQ ID NO: 75, CDR2 as set forth in SEQ ID NO: 58, and CDR3 as set forth in SEQ ID NO: 95;
(20) CDR1 as set forth in SEQ ID NO: 45, CDR2 as set forth in SEQ ID NO: 52, and CDR3 as set forth in SEQ ID NO: 47;
(21) CDR1 as set forth in SEQ ID NO: 63, CDR2 as set forth in SEQ ID NO: 55, and CDR3 as set forth in SEQ ID NO: 65;
(22) CDR1 as set forth in SEQ ID NO: 108, CDR2 as set forth in SEQ ID NO: 88, and CDR3 as set forth in SEQ ID NO: 101;
(23) CDR1 as set forth in SEQ ID NO: 87, CDR2 as set forth in SEQ ID NO: 91, and CDR3 as set forth in SEQ ID NO: 62;
(24) CDR1 as set forth in SEQ ID NO: 90, CDR2 as set forth in SEQ ID NO: 94, and CDR3 as set forth in SEQ ID NO: 104;
(25) CDR1 as set forth in SEQ ID NO: 102, CDR2 as set forth in SEQ ID NO: 97, and CDR3 as set forth in SEQ ID NO: 59;
(26) CDR1 as set forth in SEQ ID NO: 84, CDR2 as set forth in SEQ ID NO: 103, and CDR3 as set forth in SEQ ID NO: 107;
(27) CDR1 as set forth in SEQ ID NO: 99, CDR2 as set forth in SEQ ID NO: 106, and CDR3 as set forth in SEQ ID NO: 110;
(28) CDR1 as set forth in SEQ ID NO: 105, CDR2 as set forth in SEQ ID NO: 109, and CDR3 as set forth in SEQ ID NO: 113;
(29) CDR1 as set forth in SEQ ID NO: 78, CDR2 as set forth in SEQ ID NO: 58, and CDR3 as set forth in SEQ ID NO: 68;
(30) CDR1 as set forth in SEQ ID NO: 96, CDR2 as set forth in SEQ ID NO: 112, and CDR3 as set forth in SEQ ID NO: 115;
(31) CDR1 as set forth in SEQ ID NO: 48, CDR2 as set forth in SEQ ID NO: 114, and CDR3 as set forth in SEQ ID NO: 117;
(32) CDR1 as set forth in SEQ ID NO: 72, CDR2 as set forth in SEQ ID NO: 64, and CDR3 as set forth in SEQ ID NO: 74.

2. The anti-TfR1 single domain antibody of claim 1, wherein the amino acid sequence of a VHH chain of the anti-TfR1 single domain antibody is selected from one or more of SEQ ID NOs: 1-44 and 210-215.

3. An anti-TfR1 antibody comprising one or more VHH chains of the anti-TfR1 single domain antibody of claim 1 or 2.

4. A chimeric antigen receptor (CAR) comprising an extracellular domain, wherein the extracellular domain comprises the anti-TfR1 single domain antibody of claim 1 or 2, or the anti-TfR1 antibody of claim 3.

5. A multispecific antibody, wherein a first antigen-binding region of the multispecific antibody comprises the anti-TfR1 single domain antibody of claim 1 or 2, or the anti-TfR1 antibody of claim 3.

6. A recombinant protein, having:
(i) the anti-TfR1 single domain antibody of claim 1 or 2, the anti-TfR1 antibody of claim 3, or an active fragment thereof;
(ii) optionally, a polypeptide molecule or fragment with therapeutic function; and/or
(iii) optionally, a functional domain that improves the physicochemical properties or drugability of the protein.

7. A polynucleotide encoding a protein selected from the group consisting of: the anti-TfR1 single domain antibody of claim 1 or 2, the anti-TfR1 antibody of claim 3, the chimeric antigen receptor of claim 4, the multispecific antibody of claim 5, the recombinant protein of claim 6, and a combination thereof.

8. An expression vector comprising the polynucleotide of claim 7.

9. A host cell comprising the expression vector of claim 8, or having the polynucleotide of claim 7 integrated into its genome.

10. An immunoconjugate, comprising:
(a) the anti-TfR1 single domain antibody of claim 1 or 2, the anti-TfR1 antibody of claim 3, the multispecific antibody of claim 5, or the recombinant protein of claim 6; and
(b) a conjugated moiety selected from the group consisting of: a detectable label, a drug, a cytokine, a radionuclide, an enzyme, a gold nanoparticle/nanorod, a magnetic nanoparticle, a viral capsid protein, a virus-like particle (VLP), an oligonucleotide, a nucleic acid analog, and a combination thereof.
